# EUROPEAN PATENT APPLICATION

(11) **EP 3 222 712 A1**
(43) Date of publication of application: **27.09.2017**
(21) Application number: 16000685.4
(22) Date of filing: 22.03.2016
(51) Int. Cl.: C12N 9/02

(54) **ALCOHOL DEHYDROGENASE FROM PICHIA PASTORIS AND USE THEREOF**

(71) Applicant: Universität zu Köln, 50923 Köln (DE); Universität Bielefeld, 33615 Bielefeld (DE)
(72) Inventor: Hummel, Werner, 52445 Titz (DE); Gröger, Harald, 33739 Bielefeld (DE); Berkessel, Albrecht, 50374 Erftstadt (DE); Bulut, Dalia, 41061 Mönchengladbach (DE)
(74) Representative: Wilk, Hans-Christoph

(57) **Abstract**

The present invention relates to a new alcohol dehydrogenase from saccharomycetales, particularly from *Pichia pastoris,* nucleic acids encoding the alcohol dehydrogenase, methods of producing the alcohol dehydrogenase, and the use of the alcohol dehydrogenase for producing enantiomer-enriched organic compounds, such as alcohols and particularly diols.

SEQUENCE LISTING

<110>  UNIVERSITÄT ZU KÖLN, UNIVERSITÄT BIELEFELD

<120>  Alcohol dehydrogenase from Pichia pastoris and use thereof

<130>  UD 40753 / SAM

<160>  5

<170>  PatentIn version 3.3

<210>  1
<211>  1065
<212>  DNA
<213>  pichia pastoris

<400>  1
atggtttcta aggttttatt gacaggtgct tctggttaca tcgcccaaca catcactaat      60

gaattacttt cccacggctt taaggtcatt ggaactgtaa gaagacagga gcaggcagac     120

caattgcata agcaattttc agaggaaagt tctgttttac aaaaagatcc ctcccttctg     180

acctatgtcc ttgtcccaga tattggtgct tcggatgctt ttgatgaggt attgaagtca     240

acccccgaca tcacttatgt ccttcacaca gcttccccat ttattttcaa tgacgacaga     300

gctcttgagg acgtttattt gaagcctgct gttgagggta ccagaaacat cttaagtgct     360

atcaagaagt ttgccaatga cagtgtcaag aacgtcgtgg taacttcctc ctttgcagca     420

attttgaacg ctgacaagtt tgaagacaag agtttcattc acactgagaa ggtgtggaat     480

aacaacacct gggatcaaac caagagtgga gacagaggtg ttgcttacat cgtttccaag     540

aaggaggctg agaaagctgc ctgggacttc gttgagaagg agaaaccaaa ctttaaattg     600

acaacagtaa accctcctta tgtttttggt cctcagaaat tcgatgcttc ggctaagaag     660

gaatctttga acacctcagc tgagatcgtt ggatctctgt tgcacacaaa gtatccatct     720

gatgacaagt tgtttgatga tcccctcaat ttatccgttg atgttagaga cgtagctctc     780

taccatgttc tgccactttt gaatgctgat ttggcttcca ggagattgct tgttgttcaa     840

tccaagttca gcgctcagag aatcctgaac attatcaacg agaattttcc tgaattaaag     900

ggcaagatcg cagttggaaa gcccgaggaa acggcaagag tggaagctat taagggcccg     960

gagtataaca acagtgttac tgtaggccta actggagttg accccatccc actggagaaa    1020

acggttgtgg actctgtcaa acagatattg cgtgctaata aataa                    1065

<210>  2
<211>  354
<212>  PRT
<213>  Pichia pastoris

<400>  2

Met Val Ser Lys Val Leu Leu Thr Gly Ala Ser Gly Tyr Ile Ala Gln

1               5                   10                  15

His Ile Thr Asn Glu Leu Leu Ser His Gly Phe Lys Val Ile Gly Thr
            20                  25                  30

Val Arg Arg Gln Glu Gln Ala Asp Gln Leu His Lys Gln Phe Ser Glu
        35                  40                  45

Glu Ser Ser Val Leu Gln Lys Asp Pro Ser Leu Leu Thr Tyr Val Leu
    50                  55                  60

Val Pro Asp Ile Gly Ala Ser Asp Ala Phe Asp Glu Val Leu Lys Ser
65                  70                  75                  80

Thr Pro Asp Ile Thr Tyr Val Leu His Thr Ala Ser Pro Phe Ile Phe
                85                  90                  95

Asn Asp Asp Arg Ala Leu Glu Asp Val Tyr Leu Lys Pro Ala Val Glu
            100                 105                 110

Gly Thr Arg Asn Ile Leu Ser Ala Ile Lys Lys Phe Ala Asn Asp Ser
        115                 120                 125

Val Lys Asn Val Val Val Thr Ser Ser Phe Ala Ala Ile Leu Asn Ala
    130                 135                 140

Asp Lys Phe Glu Asp Lys Ser Phe Ile His Thr Glu Lys Val Trp Asn
145                 150                 155                 160

Asn Asn Thr Trp Asp Gln Thr Lys Ser Gly Asp Arg Gly Val Ala Tyr
                165                 170                 175

Ile Val Ser Lys Lys Glu Ala Glu Lys Ala Ala Trp Asp Phe Val Glu
            180                 185                 190

Lys Glu Lys Pro Asn Phe Lys Leu Thr Thr Val Asn Pro Pro Tyr Val
        195                 200                 205

Phe Gly Pro Gln Lys Phe Asp Ala Ser Ala Lys Lys Glu Ser Leu Asn
    210                 215                 220

Thr Ser Ala Glu Ile Val Gly Ser Leu Leu His Thr Lys Tyr Pro Ser
225                 230                 235                 240

Asp Asp Lys Leu Phe Asp Asp Pro Leu Asn Leu Ser Val Asp Val Arg
                245                 250                 255

Asp Val Ala Leu Tyr His Val Leu Pro Leu Leu Asn Ala Asp Leu Ala
            260                 265                 270

Ser Arg Arg Leu Leu Val Val Gln Ser Lys Phe Ser Ala Gln Arg Ile
        275                 280                 285

Leu Asn Ile Ile Asn Glu Asn Phe Pro Glu Leu Lys Gly Lys Ile Ala
    290                 295                 300

Val Gly Lys Pro Glu Glu Thr Ala Arg Val Glu Ala Ile Lys Gly Pro
305                 310                 315                 320

Glu Tyr Asn Asn Ser Val Thr Val Gly Leu Thr Gly Val Asp Pro Ile
                325                 330                 335

Pro Leu Glu Lys Thr Val Val Asp Ser Val Lys Gln Ile Leu Arg Ala
            340                 345                 350

Asn Lys

<210>  3
<211>  6
<212>  PRT
<213>  artificial

<220>
<223>  His tag

<400>  3

His His His His His His
1               5

<210>  4
<211>  23
<212>  DNA
<213>  Artificial

<220>
<223>  primer

<400>  4
gcgcttccat atggtttcta agg                                              23

<210>  5
<211>  26
<212>  DNA
<213>  Artificial

<220>
<223>  primer

<400>  5

cggctcgaga ttatttatta gcacgc 26

## Description

The present invention relates to the technical fields of industrial microbiology and alcohol production. Specifically, the invention relates to an alcohol dehydrogenase from saccharomycetales, particularly from *Pichia pastoris,* as well as the nucleic acids coding for this enzyme and vehicles containing the nucleic acids. In particular the invention relates to an alcohol dehydrogenase from *Pichia pastoris strain* GS 115.

Alcohol dehydrogenases catalyze a multiplicity of biological reactions, with alcohol substrates being oxidized to the corresponding ketones or aldehydes or, in opposite direction, the reduction of the aldehyde or ketone to the alcohol being catalyzed. The reactions described are usually reversible and take place in the presence of nicotinamide adenine dinucleotide (NAD⁺ / NADH) or nicotinamide adenine dinucleotide phosphate (NADP⁺ / NADPH) as co-enzyme (co-factor).

Besides the outstanding importance of alcohol dehydrogenases in biological processes, these enzymes are also regarded as interesting catalysts for the organo-chemical synthesis of alcohols, ketones or aldehydes. The biocatalytic synthesis of optically active compounds such as alcohols, hydroxy aldehydes or hydroxy ketones becomes more important. A special interest is in biocatalysts that enable regio-selective reactions. The coupled use of two dehydrogenases under regeneration of cofactors or the substrate-coupled coenzyme UD 40753 / SAM:AL regeneration has proven as a possible method for the commercial synthesis of such compounds as disclosed in W. Hummel, Adv. Biochem. Engineering / Biotechnology 1997, 58, 145-184).

Limiting for the technical application of alcohol dehydrogenases are the substrate spectra of the enzymes. Each enzyme only converts a limited spectrum of substrates. The enzymes moreover show characteristic regio and stereo selectivity, which also limit their use. In addition, only a modest number of alcohol dehydrogenases is available in a recombinant form required for industrial purposes.

There is therefore great interest in finding further alcohol dehydrogenases having novel enzymatic properties, in particular regarding the reduction of 1-hydroxy-3-ketones for the synthesis of 1,3-diols. The following scheme 1 illustrates the reduction of a 1-hydroxy-3-ketone (1) to a 1,3-diol (2) wherein R=CF₃ or CH₃. 1,3-Diols at the moment gain much interest in organic chemistry as components in pharmaceuticals or as precursor of fragrances.

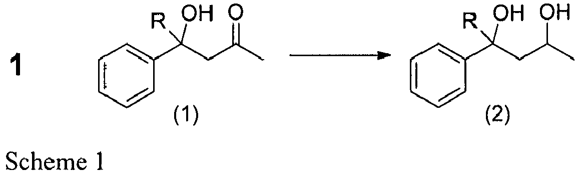

It is therefore the object of the present invention to indicate a further alcohol dehydrogenase which catalyses the reduction of 1-hydroxy-3-ketones.

The object is achieved according to the claims. The object particularly is achieved by a nucleic acid coding for an alcohol dehydrogenase according to SEQ ID NO: 1 or the sequence complementary thereto, and an alcohol dehydrogenase encoded by the nucleic acid, particularly an alcohol dehydrogenase according to SEQ ID NO:2.

The term "nucleic acid" as used herein includes single-strand or double-strand DNA as well as RNA or mixtures thereof.

The nucleic acid sequences of the invention surprisingly enable recombinant alcohol dehydrogenases of the type *Pichia pastoris* to be obtained, which can be expressed in host organisms such as, for example, *Escherichia coli.* The alcohol dehydrogenase is able to reduce also sterically demanding aldehydes such as benzaldehyde with an unusually high activity and to reduce the hydroxyketone according to formula (3) below to the respective 1,3-diol with high selectivity. The stability of the alcohol dehydrogenases obtained in this way also enables a use on an industrial scale that is particularly advantageous from an economic point of view.

Using the polypeptides having alcohol dehydrogenase activity encoded by the nucleic acids it is possible to reduce stereoselectively the (S)-enantiomer of the racemic hydroxyketone (3) to the 1,3-diol and thus obtain the enantiomer-enriched (R)-hydroxyketone, as is shown the the following scheme 2:

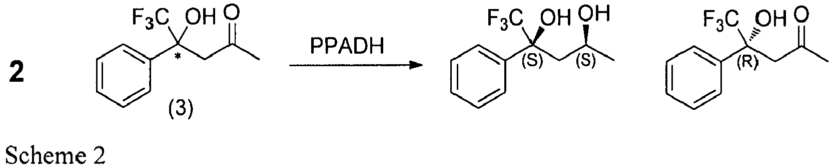

The enzyme according to the invention particularly catalyses the reaction shown in the following scheme 3:

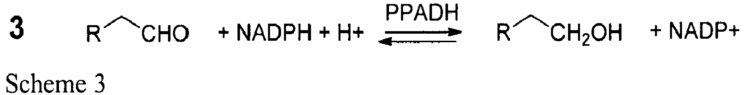

The nucleic acid coding for an alcohol dehydrogenase according to SEQ ID NO:1 or a sequence complementary thereto advantageously catalyses enantio-selective and stereoselective reactions. The alcohol dehydrogenases advantageously catalyses the reaction in the presence of nicotinamide adenine dinucleotide (NAD) or nicotinamide adenine dinucleotide phosphate (NADP). For these co-enzymes methods for regeneration are well-known. Apart from this, also specific ketones such as the compound (1) as shown in scheme 1 are reduced or secondary alcohols are oxidised, respectively.

Further isolated nucleic acids based on the sequence of SEQ ID NO: 1 may prove advantageous, particularly the following nucleic acid sequences coding for a polypeptide having alcohol dehydrogenase activity selected from the group consisting of:
a) a nucleic acid sequence hybridizing with the nucleic acid sequence according to SEQ ID NO: 1 or the sequence complementary thereto under stringent conditions,
b) a nucleic acid sequence having a homology of at least 75% to SEQ ID NO: 1 or to the sequence complementary to SEQ ID NO: 1, and
c) a nucleic acid sequence coding for a polypeptide having improved activity and/or selectivity and/or stability compared to the polypeptide of SEQ ID NO: 1, prepared by
   i) mutagenesis of SEQ ID NO: 1,
   ii) cloning of the nucleic acid sequence obtainable from i) into a suitable vector with suitable transformation into a suitable expression system, and
   iii) detection of the polypeptide in question having improved activity and/or selectivity and/or stability.

The present disclosure also relates, in addition to the nucleic acid sequences of SEQ. ID. NO: 1, also to those which hybridize with the nucleic acid sequence of the invention or its complementary sequence under stringent conditions, and other nucleic acid sequences which have been improved by suitable mutagenesis processes. More specifically, those nucleic acids are also comprised which are alleles or functional variants of the nucleic acid sequences of the invention. Functional variants preferably are more than 80%, more preferably more than 85%, 90%, and particularly preferably more than 95%, homologous to SEQ. ID. NO: 1.

Procedures of improving the nucleic acid sequences of the invention or the polypeptides encoded by them by using mutagenesis methods are known to a person skilled in the art. Suitable mutagenesis methods available to the person skilled in the art particular are saturation mutagenesis, random mutagenesis, in vitro recombination methods and site-directed mutagenesis (Eigen, M. and Gardiner, W., Evolutionary molecular engineering based on RNA replication, Pure Appl. Chem. 1984, 56, 967-978; Chen, K. and Arnold, F., Enzyme engineering for nonaqueous solvents: random mutagenesis to enhance activity of subtilisin E in polar organic media. Bio/Technology 1991, 9, 1073-1077; Horwitz, M. and Loeb, L., Promoters Selected From Random DNA-Sequences, Proc Natl Acad Sci USA 83, 1986, 7405-7409; Dube, D. and L. Loeb, Mutants Generated By The Insertion Of Random Oligonucleotides Into The Active-Site Of The Beta-Lactamase Gene, Biochemistry 1989, 28, 5703-5707; Stemmer, P. C, Rapid evolution of a protein in vitro by DNA shuffling, Nature 1994, 370, 389-391 and Stemmer, P. C, DNA shuffling by random fragmentation and reassembly: In vitro recombination for molecular evolution. Proc Natl Acad Sci USA 91, 1994, 10747-10751). The new nucleic acid sequences obtained can be cloned into a host organism according to the methods indicated herein below and the polypeptides expressed in this way can be detected using suitable screening methods and subsequently isolated.

The present disclosure also relates to nucleic acid sequences which hybridize with the single-stranded nucleic acid sequences of the invention or their complementary single-stranded nucleic acid sequences under stringent conditions. The term "under stringent conditions" here has the meaning that a hybridization is stringent when a positive hybridization signal is observed after washing with 1 x SSC (150 mM sodium chloride, 15 mM sodium citrate, pH 7.0) and 0.1% SDS (sodium dodecyl sulphate) at 50 °C, preferably at 55 °C, more preferably at 62 °C and most preferably at 68 °C for 1 hour, and more preferably with 0.2 x SSC and 0.1% SDS at 50 °C, more preferably at 55 °C, more preferably at 62 °C and most preferably at 68 °C for one hour.

The information of SEQ. ID. NO: 1 may be utilized for generating primers in order to identify and to clone allelic forms by means of PCR, for example in other Pichia strains. In addition it is possible, owing to the sequence information, to utilize probes for finding further naturally occurring functional variants of the nucleic acids of the invention and thus the corresponding encoded enzyme variants. Starting from SEQ. ID. NO: 1 or from functional variants which are allelic thereto or occur naturally, a library of artificially generated functional enzyme variants may be obtained, for example via PCR by using a faulty DNA polymerase.

A further aspect relates to an alcohol dehydrogenase from saccharomycetales, particularly selected from the group comprising *Pichia pastoris, Wickerhamomyces ciferrii, Cyberlindnera jadinii, Kluyveromyces marxianus, Ogataea parapolymorpha, Kuraishia capsulata, Lachancea quebecensis, Geotrichum candidum, Spathaspora passalidarum, Saccharomyces cerevisiae,* and *Chlamydia trachomatis.* Particularly, the invention relates to an alcohol dehydrogenase from *Pichia pastoris,* in particular an alcohol dehydrogenase from *Pichia pastoris* GS 115. In a preferred embodiment, the alcohol dehydrogenase is encoded by a nucleic acid according to SEQ ID NO:1 or the sequence complementary thereto. In another preferred embodiment, the alcohol dehydrogenase has a sequence according to SEQ ID NO:2, or the alcohol dehydrogenase has at least 80% sequence identity to SEQ ID NO: 2. These polypeptides can be employed very well in industrial processes owing to their regio- and stereo-selectivity and expanded substrate spectrum.

As used herein, the term "polypeptide" refers to a chain of amino acids linked to one another by a peptide bond. A polypeptide may include a chain of at least 6 amino acids, in other embodiments a polypeptide may include a long, continuous peptide chain which also may be referred to as a protein. As used herein, the term "enzyme" refers to a polypeptide or protein that is capable of catalyzing a chemical reaction, such as, for example, alcohol dehydrogenases catalyze the oxidation of alcohol substrates to the corresponding ketones or aldehydes or the reduction of an aldehyde or ketone to an alcohol. If not stated otherwise, the term polypeptide may be used synonymous to the term protein or enzyme to refer to the alcohol dehydrogenases of the invention.

The invention also refers to the allelic or functional variants of the alcohol dehydrogenases of the invention. A functional variant means for the purposes of the present invention an alcohol dehydrogenase whose amino acid sequence has more than 80%, preferably more than 85%, 87%, and more preferably more than 90%, sequence identity to SEQ. ID. NO: 2. It is possible to introduce by mutagenesis amino acid substitutions into a polypeptide, without the activity and/or selectivity and/or stability of the polypeptide being reduced substantially compared to the polypeptide of SEQ. ID. NO: 2. Thus, for example, amino acids which are not located at the active site and whose replacement by an amino acid of the same group is not expected to result in a substantially altered three-dimensional structure may be replaced by an amino acid of the same group. For example, particular amino acids having non-polar side chains such as alanine and valine can be expected to be replaced by each other, without causing a (substantial) influence on the catalytic or biochemical function of the enzyme of the invention. On the basis of his expertise, a person skilled in the art knows or can draw corresponding conclusions on the substitution of other types of amino acids such as the replacement of acidic amino acids by other acidic amino acids. The polypeptides having alcohol dehydrogenase activity (alcohol dehydrogenases) according to the invention may additionally comprise post-translational modifications such as glycosylations or phosphorylations.

The alcohol dehydrogenases may comprise at least one heterologous amino acid sequence which characterizes these polypeptides as fusion proteins. As used herein, the term "fusion protein" refers to a sequence of amino acids, wherein a part of the sequence is derived from one origin and fused to another part of the sequence that is derived from one or more other origin. The origin of a protein or an amino acid sequence may be native or synthetic, such as for a tag. This term refers to the original source of the sequences, as in practice when the fusion protein is prepared by recombinant techniques there is no distinction between the fused parts. Examples of heterologous components may be tags, e.g. a His tag or Flag tag, which may be employed in the purification of the fusion proteins of the invention. In other embodiments, heterologous components may have a separate enzymatic activity. In such a case, the two enzymatic components are preferably connected by a linker such as a flexible glycine or glycine-serine linker of 6-10 amino acids in length, in order to ensure functionality of the components. As used herein, the term "heterologous" may mean on the one hand that the components of the fusion protein do not naturally occur covalently linked together, and on the other hand that the components come from different species. Fusion proteins are usually prepared by recombinant DNA technology.

In a preferred embodiment, the alcohol dehydrogenase comprises at least one N-terminal and/or C-terminal His tag. As used herein, the term "His tag" or "polyhistidine-tag" refers to an amino acid motif comprising at least six histidine (His) residues. The total number of histidine residues of the tag may vary. The His tag may have a sequence of 6 to 10 histidines. Preferably, the His tag is a 6xHis tag HHHHHH (SEQ ID NO: 3). Advantageously, the His tag does not appear to interfere with the bioactivity or the biodistribution of the fusion protein. Surprisingly it was found that a fusion of the alcohol dehydrogenase with an N-terminal His tag advantageously increased the enzymatic activity in reducing benzaldehyde by nearly 95% compared to the wild-type enzyme.

In a further aspect, the present invention relates to recombinant expression systems or recombinant plasmids and vectors comprising one or more of the nucleic acids of the invention. As used herein, the term "vector" refers to discrete elements that are used to introduce heterologous DNA into cells for either expression or replication thereof. Selection and use of such vehicles are well known within the skill of the artisan. An expression vector may be a recombinant DNA construct, such as a plasmid, that, upon introduction into an appropriate host cell, results in expression of a cloned DNA.

An expressions system means a system for a recombinant expression of the nucleic acids of the invention and thus for the recombinant production of the polypeptides of the invention. This production may preferably take place in microorganisms or other hosts transformed or transfected with corresponding nucleic acid sequences or vectors. The terms "transformation" and "transfection" are used in the same sense according to the present invention.

In a further aspect, the present invention relates to a microorganism comprising a plasmid comprising one or more of the nucleic acids of the invention. The recombinant microorganism is preferably of prokaryotic origin. Suitable host cells include cells of monocellular microorganisms such as bacterial cells. Suitable microorganisms in this respect are prokaryotes such as *E. coli, Bacillus subtilis* or *Pseudomonas sp.* Other bacteria which may be used for expression of the nucleic acid sequences of the invention are those of the genera/species *Lactobacillus, Bacillus, Rhodococcus, Campylobacter, Caulobacter, Mycobacterium, Streptomyces, Neisseria, Ralstonia, Pseudomonas,* and *Agrobacterium.* Appropriate strains are known in the prior art, or commercially available or may, at least partially, be obtained from the international deposition sites such as American Type Culture Collection (ATCC) or the German Collection of Microorganisms and Cell Cultures (DSMZ).

Likewise eukaryotes such as mammalian cells, insect cells or plant cells or organisms such as, for example, yeasts like *Hansenula polymorphs, Pichia sp., Saccharomyces cerevisiae,* be used for recombinant production of the polypeptides. The yeast strain *Pichia sp.* is most preferred as a host strain due to the possible homologous expression of the gene in its natural environment and host, which generally yield in higher overexpression. A further aspect of the invention relates to a method for producing alcohol dehydrogenases from *Pichia pastoris* according to the invention, wherein the microorganisms are cultivated under aerobic conditions. This method has proven advantageous for producing alcohol dehydrogenases from *Pichia pastoris* (PPADH).

Methods of cloning are well-known to the person skilled in the art. Preference is given to utilizing *E. coli* strains for this purpose, particularly *E. coli* XLl Blue, DH5α, NM 522, JMlOl, JM109, JM105, RR1, DH5CC, TOP 10-, HBlOl, BL21 codon plus, BL21 (DE3) codon plus, BL21, BL21 (DE3), MM294, W3110, DSM14459 as described in EP 1444367.

Furthermore, recombinant production of the polypeptides of the invention may take place in a non-human host. Suitable eukaryotic cells include CHO cells, HeLa cells and others. Many of these cells can be obtained from deposition sites such as ATCC or DSMZ. The transformation or transfection described above may be carried out by known methods, for example by calcium phosphate co-precipitation, lipofection, electroporation, PEG/DMSO methods, particle bombardment or viral/bacteriophage infection. The cell may contain the recombinant nucleic acid in an extrachromosomal or a chromosomally integrated form. Transfection and transformation protocols are known to the skilled worker (Chan and Cohen. 1979. High Frequency Transformation of Bacillus subtilis Protoplasts by Plasmid DNA. MoI Gen Genet. 168 (1):111-5; Pigac and Schrempf, 1995, A Simple and Rapid Method of Transformation of Streptomyces rimosus R6 and Other Streptomycetes by Electroporation, Appl. Environ. Microbiol., p. 352-356; Irani and Rowe. 1997. Enhancement of transformation in Pseudomonas aeruginosa PAOl by Mg2+ and heat. Biotechniques 22: 54-56).

The host may be a transgenic non-human animal. Transgenic non-human animals may be produced by methods known in the prior art. Preferably, the transgenic non-human animal may have various genetic constitutions. It may (i) overexpress the gene of a nucleic acid sequence of the invention in a constitutive or inducible manner, (ii) contain the endogenous gene of a nucleic acid sequence according to the invention in an inactivated form, (iii) contain the endogenous gene of a nucleic acid sequence according to the invention, which has been completely or partially replaced by a mutated gene of a nucleic acid sequence according to the invention, (iv) have conditional and tissue-specific overexpression or underexpression of the gene of a nucleic acid sequence according to the invention or (v) have a conditional and tissue-specific knock-out of the gene of a nucleic acid sequence according to the invention. Preferably, the transgenic animal additionally contains an exogenous gene of a nucleic acid sequence according to the invention under the control of a promoter allowing overexpression. Alternatively, the endogenous gene of a nucleic acid sequence according to the invention may be overexpressed by activating or/and replacing its own promoter. The endogenous promoter of the gene of a nucleic acid sequence according to the invention preferably has a genetic modification resulting in increased expression of the gene. The said genetic modification of the endogenous promoter comprises both a mutation of individual bases and deletion and insertion mutations.

The host in a preferred embodiment, is a transgenic rodent, preferably a transgenic mouse, a transgenic rabbit, a transgenic rat, or is a transgenic sheep, a transgenic cow, a transgenic goat or a transgenic pig. Mice have numerous advantages over other animals. They are easy to keep and their physiology is regarded as a model system for that of humans. The generation of such gene- manipulated animals is sufficiently known to the skilled worker and is carried out by customary methods. Alternatively or additionally it is also possible to use cell culture systems, in particular human cell culture systems, for the applications described for the non-human transgenic animal.

The coding nucleic acid sequences may be cloned into conventional plasmids or vectors and expressed in cell culture after transfection of microorganisms or other host cells with such vectors. Suitable plasmids or vectors are in principle any embodiments available to the skilled worker for this purpose. Plasmids and vectors of this kind may be found, for example, in Studier and co-workers (Studier, W. F.; Moffatt B. A. (1986) Use of Bacteriophage T7 RNA polymerase to Direct Selective High Level Expression of Cloned Genes. J. Mol. Biol. 189, 113). Other preferred plasmids and vectors may be found in: Terpe, K. (2006) Overview of bacterial expression systems for heterologous protein production: from molecular and biochemical fundamentals to commercial systems, Appl. Microbiol. Biotechnol. 72, 211.

Plasmids which may be used for cloning the gene construct having the nucleic acid sequence according to the invention into the host organism in a preferred manner are: pUC18 (Roche Biochemicals), pKK-177-3H (Roche Biochemicals), pBTac2 (Roche Biochemicals), pKK223-3 (Amersham Pharmacia Biotech), pKK-233-3 (Stratagene) or pET (Novagen). Suitable vectors are also, for example, pET-21a (+) or pET28-a(+) for *E. coli,* or pETDuet-1 (Amp^{R}) or pACYC-Duet-1 (CmR), but other expression vectors for prokaryotic unicellular organisms and vectors for eukaryotes, such as, for example, yeasts and insect or mammalian cells may also be used. Examples of vectors which have proved suitable for yeasts are the pREP vector and the pINT vector. For expression in insect cells, for example, Baculovirus vectors such as in EP127839 or EP549721 have been disclosed, and SV40 vectors which are generally obtainable, for example, are suitable for expression in mammalian cells. Particular preference is given to vectors for unicellular eukaryotic organisms, in particular from the group of pET vectors such as pET28-a(+) for transformation of *E. coli* cells.

In a preferred embodiment, the nucleic acid sequence of the invention, which has been introduced into the vector, is additionally fused to a histidine tag provided by the vector. Preference is given to cloning the introduced nucleic acid sequence into the pET-28a(+) vector so as for transcription to be under the control of the IPTG-regulatable promoter present in the vector. Alternatively, preference is also given to employing rhamnose-regulatable promoters. Besides the usual markers such as, for example, antibiotic resistance genes, the vectors may contain further functional nucleotide sequences for regulating, in particular repressing or inducing, expression of the ADH gene and/or of a reporter gene. Preference is given to utilizing as promoters regulatable weak promoters such as, for example, the rha promoter or the nmtl promoter, or regulatable strong promoters such as, for example, the lac, ara, lambda, pL, T7 or T3 promoter. The coding DNA fragments must be transcribable from a promoter in the vectors. Other examples of proven promoters are the Baculovirus polyhedrin promoter for expression in insect cells (see, for example, EP127839) or the early SV40 promoter or LTR promoters, for example, of MMTV (Mouse Mammary Tumour Virus; Lee et al. (1981) Nature, 294 (5838), 228-232). The expression vectors may contain further functional sequence regions such as, for example, an origin of replication, operators or termination signals.

In a further aspect, the invention relates to partial sequences of the nucleic acid sequences according to the invention, which partial sequences preferably consist of at least 5 or 10, preferably 50, more preferably 100, very preferably 150, contiguous nucleotides, particularly preferably of at least 300 contiguous nucleotides of the nucleic acids according to the invention. These are in particular primers for preparing the nucleic acid sequences according to the invention particularly by means of PCR or LCR, or probes for fishing the nucleic acid sequences according to the invention. Preferred are primers for producing the nucleic acids according to the invention. The primers may be derived from the 3' and 5' ends of the nucleic acid sequences according to the invention. Preferably, they additionally have common cleavage sites such as, for example, *Nde*l and *Xho*I cleavage sites. Preferred primers are selected from 5'GCGCTTCCATATGGTTTCTAAGG-3' (SEQ ID NO:4) and 5' CGGCTCGAGATTATTTATTAGCACGC 3'(SEQ ID NO:5).

Improved recombinant (rec) polypeptides having alcohol dehydrogenase activity may be prepared using a method starting from nucleic acid sequences according to the invention, wherein a) the nucleic acid sequences are subjected to a mutagenesis, b) the nucleic acid sequences obtainable from a) are cloned into a suitable vector which is transferred to a suitable expression system, and c) the produced polypeptides having improved activity and/or selectivity and/or stability are detected and isolated. The disclosure likewise relates to rec polypeptides or nucleic acid sequences encoding them, which can be obtained by such process. Preferred are mutants of the alcohol dehydrogenase having a modified specificity for co-enzymes, particularly these mutants which accept NAD instead ofNADP as co-enzyme.

The recombinant polypeptides of the invention are usable for the enzymatic reduction of carbonyl groups, such as aldehydes and ketones, and for the enzymatic oxidation of alcohols. Aldehydes and ketones may be selected from the group of substrates as given in Table 2. Alcohols may be selected from the group of substrates as given in Table 3.

The alcohol dehydrogenase catalyses reactions preferably in aqueous solvents, which may be buffered accordingly. In embodiments, the alcohol dehydrogenase particularly is usable in a range of pH 4 to 8, preferably in a range of pH 6 to pH 8 The alcohol dehydrogenase advantageously exhibits high activity in a broad pH range which is particularly advantageous for a commercial use as catalyst in the preparation of alcohols. In further embodiments, the alcohol dehydrogenase particularly is usable in a temperature range from 20°C to 60°C, particularly in the range from 20°C to 45°C. The alcohol dehydrogenase advantageously exhibits high enzymatic activity in these temperature ranges.

The recombinant polypeptides particularly are usable for preparing alcohols, in particular enantiomerically enriched alcohols, diols, hydroxyketones, hydroxy esters or hydroxyaldehydes. A further aspect of the invention relates to the use of the alcohol dehydrogenase according to the invention for producing enantiomer-enriched or diastereomer-enriched organic compounds, such as alcohols. The organic compounds preferably are selected from alcohols, diols, hydroxyketones, hydroxy esters or hydroxyaldehydes, particularly diols, most preferred 1,3-diols. The alcohol dehydrogenase is usable for the enantiopure and diastereomerically pure synthesis of alcohols such 1,3-diols. Further the alcohol dehydrogenase is usable for the kinetic resolution of racemic alcohols, ketones or aldehydes. By enzymatically converting or consuming one enantiomer of a racemat, the other enantiomer will be enriched.

A chiral organic compound comprises at least one stereocenter in the molecule which gives rise to the existence of optical isomers of the compound. The term "enantiomer-enriched" compounds within the scope of the invention is understood to mean the presence of an optical antipode in a mixture with the other antipode, in an amount greater than 50 mol %. Enantiomers may be denoted according to the *R* / *S* system.

In a preferred embodiment, producing chiral enantiomer-enriched or diastereomer-enriched organic compounds is performed using a cofactor regenerating system. The system preferably comprises a cofactor and an enzyme for regenerating the cofactor. Suitable co factors for the alcohol dehydrogenase are nicotinamide adenine dinucleotide (NAD⁺ / NADH) or nicotinamide adenine dinucleotide phosphate (NADP⁺ / NADPH). An enzyme system suitable for regenerating NADPH or NADH preferably is selected from the group comprising a glucose dehydrogenase, formate dehydrogenase, or a second alcohol dehydrogenase in combination with an appropriate cosubstrate such as glucose, formate, or an alcohol. For regenerating NADP- or NAD enzymes such as NAD(P)H oxidase, lactate dehydrogenase or glutamate dehydrogenase can be applied together with an appropriate cosubstrate such as O₂, pyruvate or ketoglutarate (for a review concerning regeneration methods for nicotinamide coenzymes see for example Weckbecker, A. et al. (2010) Adv. Biochem. Engin. /Biotechnol. 120, 195-242).

In embodiments, a co-substrate is used for regenerating the co-factor, preferably a primary or secondary alcohol. Primary alcohols usable as a co-substrate may be selected from methanol, ethanol, long-chain or aromatic alcohols. Secondary alcohols usable as a co-substrate preferably are selected from the group comprising long-chain aliphatic or aromatic alcohols. The term "long-chain" alcohols as used herein refers to alcohols having in a range of 4-6 to 22-26 carbon atoms. Long-chain alcohols may be derived from natural sources, preferred long-chain alcohols are lauryl and stearyl alcohols.

A further aspect of the invention relates to the use of the nucleic acid sequences of the invention for preparing whole-cell catalysts, and to the use of the nucleic acid sequences of the invention for mutagenesis. The invention further relates to a whole-cell catalyst, comprising a nucleic acid according to the invention particularly of SEQ. ID. NO: 1. The whole-cell catalyst preferably comprises a cloned gene coding for a polypeptide having alcohol dehydrogenase activity according to the invention, and a cloned gene for an enzyme suitable for regenerating NADPH, or NADH in the case of NAD-depending mutants. The enzyme and enzyme system suitable for regenerating NADPH or NADH preferably is selected from the group referred to above, the group comprising a glucose dehydrogenase, formate dehydrogenase, or a second alcohol dehydrogenase in combination with an appropriate cosubstrate such as glucose, formate, or an alcohol. For regenerating NADP- or NAD enzymes such as NAD(P)H oxidase, lactate dehydrogenase or glutamate dehydrogenase can be applied together with an appropriate cosubstrate such as O₂, pyruvate or ketoglutarate. The whole-cell catalyst preferably comprises a cloned gene coding for a polypeptide having alcohol dehydrogenase activity according to the invention, and a cloned gene for a glucose dehydrogenase. The whole-cell catalyst preferably comprises a gene coding for an alcohol dehydrogenase from saccharomycetales, particularly selected from the group comprising *Pichia pastoris, Wickerhamomyces ciferrii, Cyberlindnera jadinii, Kluyveromyces marxianus, Ogataea parapolymorpha, Kuraishia capsulata, Lachancea quebecensis, Geotrichum candidum, Spathaspora passalidarum, Saccharomyces cerevisiae,* and *Chlamydia trachomatis.* Particularly, the whole-cell catalyst preferably comprises a gene coding for an alcohol dehydrogenase from *Pichia,* preferably from *Pichia pastoris,* more preferably from *Pichia pastoris* GS115, in particular one that codes for the alcohol dehydrogenase of the invention according to SEQ. ID. NO: 2. The advantage of an organism of this kind is simultaneous expression of both polypeptide systems, particularly an alcohol dehydrogenase and an enzyme suitable for regenerating the co-enzyme, thus requiring growing only one recombinant organism for the reaction. In embodiments, the whole-cell catalyst may comprise a bacterial cell such as *E. coli* transformed with a plasmid comprises a cloned gene coding for a polypeptide having alcohol dehydrogenase activity according to the invention, and a cloned gene for a glucose dehydrogenase.

In order to adjust expression of the polypeptides with regard to their conversion rates, the correspondingly encoding nucleic acid sequences may be put on different plasmids with different copy numbers and/or promoters with different strengths may be used for expressing the nucleic acid sequences at different levels. Advantageously, a possibly inhibitory intermediate is not accumulated in enzyme systems adjusted in this way, and the observed reaction may proceed with an optimal overall rate. This is sufficiently known to the skilled worker (Gellissen, G.; Piontek, M.; Dahlems, U.; Jenzelewski, V. ; Gavagan, J. W.; DiCosimo, R.; Anton, D. L.; Janowicz, Z. A. (1996), Recombinant Hansenula polymorpha as a biocatalyst. Coexpression of the spinach glycolate oxidase (GO) and the S. cerevisiae catalase T (CTTl) gene, Appl. Microbiol. Biotechnol. 46, 46-54; Farwick, M.; London, M.; Dohmen, J.; Dahlems, U.; Gellissen, G.; Strasser, A. W.; and DE 19920712). Thus, the nucleic acid sequences of the invention can preferably be employed for preparing recombinant polypeptides. Recombinant techniques which are sufficiently known to the skilled worker produce organisms which are capable of making available the contemplated polypeptide in an amount adequate for an industrial process. The rec polypeptides of the invention are produced by genetic engineering processes known to the skilled worker. With respect to general procedures such as PCR, cloning, expression etc., reference may also be made to the following literature and the citations therein: Universal GenomeWalker™ Kit User Manual, Clontech, 3/2000 and citations therein; Triglia T.; Peterson, M. G. and Kemp, D.J. (1988), A procedure for in vitro amplification of DNA segments that lie outside the boundaries of known sequences, Nucleic Acids Res. 16, 8186.

The invention also relates to a coupled enzymatic reaction system comprising a co-factor-dependent enzymatic transformation of a substrate with a polypeptide according to the invention and an enzymatic regeneration of the co-factor such as NAD(P)H. Advantageously, enzymatic regeneration of the co-factor may be carried out using the enzymes discussed above in connection with the whole-cell catalyst, but may also be carried out electrochemically or by chemical oxidation without the use of enzymes. A reaction system may mean any vessel in which the reaction according to the invention can be carried out, i.e. reactors of any kind such as loop reactor, stirred tank, enzyme membrane reactor etc., or diagnostic kits in any form. When using a glucose dehydrogenase, the co-factor preferably is regenerated using β-D-glucose as reductant. Alternatively, however, it is also possible to use other enzymatic or substrate-based co-factor-regenerating systems such as NADH oxidase or formate dehydrogenase.

A use of the polypeptides having alcohol dehydrogenase activity according to the invention or of the whole-cell catalyst according to the invention relates to their utilization in a process for stereoselectively reducing aldehydes or ketones.

A further aspect of the invention refers to a method for producing diols, the method comprising the step of a reduction of a ketone or an aldehyde catalyzed by the alcohol dehydrogenase or the whole-cell catalyst according to the invention. In embodiments, the method comprises the step of an aldol reaction of acetone and (trifluoro)acetophenone or its derivatives, followed by the step of reducing the reaction product catalyzed by the alcohol dehydrogenase or the whole-cell catalyst according to the invention. In further embodiments, the (trifluoro)acetophenone derivative is selected from the group comprising 4'-chloro-2,2,2-trifluoroacetophenone, 4'-bromo-2,2,2-trifluoroacetophenone, 4'-fluoro-2,2,2-trifluoroacetophenone, 4'-methyl-2,2,2-trifluoroacetophenone, 4'-methoxy-2,2,2-trifluoroacetophenone, 4'-(trifluoromethyl)-2,2,2-trifluoroacetophenone, and 2'-methoxy-2,2,2-trifluoroacetophenone, particularly 2,2,2-trifluoroacetophenone.

The aldol reaction of acetone and 2,2,2-trifluoroacetophenone or its derivatives preferably is an organo-catalytic aldol reaction. Preferably, the alcohol dehydrogenase is provided as a whole-cell catalyst. Thus, the alcohol dehydrogenase may provide for a biocatalytic reduction of the aldol.

Suitable for the enzymatic transformations of aldehydes and ketones by the alcohol dehydrogenase are aqueous solvents, which may be buffered accordingly. The reduction of aldehydes or ketones or the production of diols is preferably carried out at a temperature of between 10°C and 85°C, or in a temperature range from 20°C to 60°C, more preferably between 20°C and 50°C, most preferably in the range from 25°C to 45°C. The alcohol dehydrogenase advantageously exhibits high activity in these temperature ranges. The alcohol dehydrogenase also advantageously exhibits high activity in a broad pH range. The preferred pH for the enzymatically catalyzed reduction of carbonyl compounds with the alcohol dehydrogenase according to the invention is in a range of pH 4 to pH 9, or in a range of pH 4 to 8, more preferably between pH 5.5 and pH 8, most preferably in a range of pH 6 to pH 8.

The polypeptide may be used in its native form as homogeneously purified compounds or as recombinantly produced enzyme for application. The recombinant polypeptide may furthermore also be used as component of an intact guest organism or in connection with the disrupted cell mass of the host organism, which has been purified to any degree. It is likewise possible to use the enzymes in immobilized form (Sharma B. P.; Bailey L. F. and Messing R. A. (1982), Immobilisierte Biomaterialien - Techniken und Anwendungen, Angew. Chem. 94, 836-852). The immobilization is advantageously carried out by way of lyophilization (Paradkar, V. M.; Dordick, J. S. (1994), Aqueous-Like Activity of alpha-Chymotrypsin Dissolved in Nearly Anhydrous Organic Solvents, J. Am. Chem. Soc. 116, 5009-5010; Mori, T.; Okahata, Y. (1997), A variety of lipi-coated glycoside hydrolases as effective glycosyl transfer catalysts in homogeneous organic solvents, Tetrahedron Lett. 38, 1971-1974; Otamiri, M.; Adlercreutz, P.; Matthiasson, B. (1992), Complex formation between chymotrypsin and ethyl cellulose as a means to solubilize the enzyme in active form in toluene, Biocatalysis 6, 291-305). Particular preference is given to lyophilization in the presence of surfactants such as Aerosol OT or polyvinylpyrrolidone or polyethylene glycol (PEG) or Brij 52 (diethylene glycol monocetyl ether) (Kamiya, N.; Okazaki, S. -Y.; Goto, M. (1997), Surfactant-horseradish peroxidase complex catalytically active in anhydrous benzene, Biotechnol. Tech. 11, 375-378). Exceeding preference is given to immobilization to Eupergit®, in particular Eupergit C® and Eupergit 250L® (Rohm) (for a review see: E. Katchalski-Katzir, D. M. Kraemer, J. MoI. Catal. B: Enzym. 2000, 10, 157). Preference is likewise given to immobilization to Ni-NTA in combination with the polypeptide which has been modified by attaching a His tag (Hexa-histidine) (Petty, K.J. (1996), Metal-chelate affinity chromatography In: Ausubel, F. M. et al. eds . Current Protocols in Molecular Biology, Vol. 2, New York: John Wiley and Sons). The use as CLECs is likewise conceivable (St. Clair, N.; Wang, Y. -F.; Margolin, A. L. (2000), Cofactor-bound cross- linked enzyme crystals (CLEC) of alcohol dehydrogenase, Angew. Chem. Int. Ed. 39, 380-383). These measures may succeed in generating from recombinant polypeptides which are rendered unstable by organic solvents those which are capable of functioning in mixtures of aqueous and organic solvents or wholly in organics.

The procedure for converting ketones with the polypeptides of the invention is preferably as follows. The polypeptides are added in the desired form, for example free, immobilized, in host organisms or as whole-cell catalyst, to the aqueous solution. The carbonyl compound, where appropriate the co-factor and where appropriate the co-factor regenerating agents are added to this mixture, while maintaining preferred temperature and pH ranges. After the conversion, the alcohol obtained may be isolated from the reaction mixture by methods such as crystallization, extraction, or chromatography.

Unless otherwise defined, the technical and scientific terms used herein have the same meaning as commonly understood by one of ordinary skill in the art to which this invention belongs.

The Examples, which follow serve to illustrate the invention in more detail but do not constitute a limitation thereof.

The figures show:
- Figure 1:: the scheme of a DL-proline-catalyzed aldol reaction starting from trifluoroacetophenone and acetone
- Figure 2:: the SDS-PAGE of the protein fractions containing alcohol dehydrogenase from *Pichia pastoris* (PPADH) after the respective purification steps of example 2.
- Figure 3:: the influence of the temperature on the expression of the soluble protein (Figure 3A), and on the specific activity of PPADH in the crude extracts of *E*. *coli* BL21(DE3) after expression at 20°C, 24°C, 30°C and 37°C (Figure 3B).
- Figure 4:: the influence of an N-terminal and C-terminal His tag on the enzymatic activity of PPADH in crude extracts.
- Figure 5:: the picture of an SDS agarosegel of purified PPADH carrying an N-terminal His tag.
- Figure 6:: the effect of pH in Figure 6A) and of temperature in Figure 6B) on PPADH enzymatic activity in the reduction of the carbonyl compounds.
- Figure 7:: the residual activity of PPADH after storage at 25°C in Figure 7A) and at 4°C in Figure 7B).
- Figure 8:: the reduction of racemic CF₃ ketone to the 1,3-diol using PPADH. The diagramm depicts the relative peak area of the (S)-ketone, the (R)-ketone and the diol versus time.
- Figure 9:: the SDS page of crude extracts of whole-cell constructs WCC1, WCC2 and WCC3 and fermentated WCC3 (FWCC3) containing PPADH and a glucose dehydrogenase.
- Figure 10:: the reduction of benzaldehyde to benzylalcohol by the whole-cell catalysts WCC1, WCC2 and WCC3, in Figures 10A), 10B) and 10C), respectively.
- Figure 11:: the reduction of the CF₃-ketone by the whole-cell catalysts WCC1, WCC2 and WCC3, in Figures 11A), 11B) and 11C), respectively.
- Figure 12:: the scheme of a synthesis combining an organo-catalytic aldol reaction of 2,2,2-trifluoroacetophenone and acetone with an enzymatic reduction of the racemic ketone 1 to a chiral 1,3-diol 2.
- Figure 13:: the one-pot reaction according to Figure 12 with the relative amounts of 2,2,2-trifluoroacetophenone, the (S)-ketone, the (R)-ketone, and the 1,3-diol during the reaction course, respectively, given as a percentage of peak area versus time.
- Figure 14:: the scheme of a one-pot dynamic kinetic resolution procedure, which provides the enantiopure 1,3-diol by using an organocatalyst (eg. Singh's catalyst) and PPADH.
- Figure 15:: the nucleic acid and protein sequences of the alcohol dehydrogenase PPADH from *Pichia pastoris.*

### Example 1: Chemical synthesis of a racemic CF₃-ketone

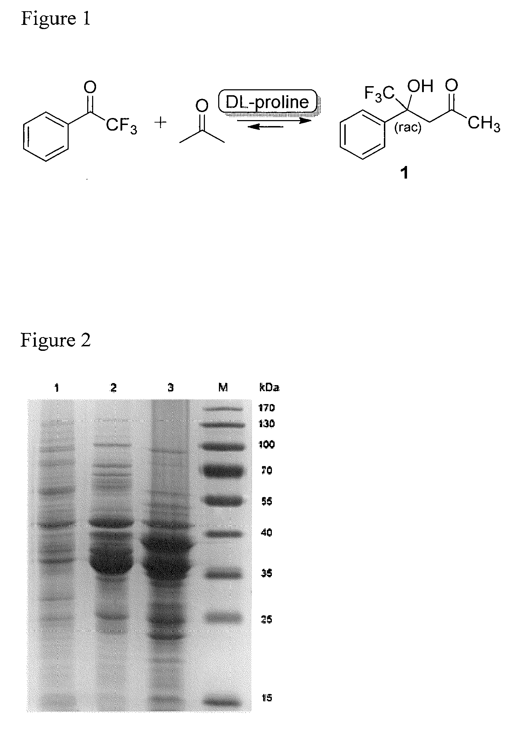

The racemic CF₃ ketone according to formula 1 as depicted in Figure 1 was synthesized via a proline-catalyzed aldol reaction. For this purpose, 25 mmol (4.35 g, 174.12 g/mol) 2,2,2-trifluoroacetophenone were poured in a 100 ml-conical flask and 2.5 mmol (0.30 g, 115.13 g/mol) of the catalyst DL-proline were added. Afterwards, 50 ml of dry acetone were added and the mixture was stirred at room temperature. After approx. 3 hours an aqueous solution was added and the mixture was extracted three times with diethyl ether and the organic phase was collected. For removing remaining water, water-free magnesium sulfate was added and the solution was filtered using a pleated filter. The filtrate was transferred to a 50 ml roundbottom flask and the solvent was evaporated at 500 bar. The remaining fluid was purged by argon and incubated on dry ice until a solid was formed. The racemic CF₃ ketone 1 was confirmed using High-performance liquid chromatography (HPLC) and Nuclear magnetic resonance spectroscopy (NMR).

The racemic CF₃ ketone according to formula 1 as depicted in Figure 1 was used as a substrate in the following examples, and generally is referred to as "the CF₃ ketone".

### Example 2: Finding, sequencing and cloning of a CF₃ ketone-converting ADH gene from Pichia pastoris GS 115

### a) Screening

Several bacteria and yeast stems obtained from the German Collection of Microorganisms and Cell Cultures (DSMZ) were cultivated under the conditions recommended by DSMZ. After 20 hours cultivation cells were centrifuged (10,000 g, 30 min, 4°C). Isolated cells were resuspended in KPi buffer (50 mM, pH 7) to yield a 25% cell suspension and macerated in a stirred cell glass bead mill (SCP disintegrator, Innomed-Konsult AB, Sweden). For this, 600 µl cell suspension were added to 1.2 g glass beads (ø 0,25 - 0,50 mm) and macerated by grinding in the stirred cell glass bead mill at 3000 rpm and 4°C for 5 minutes. The macerated cells were centrifuged off at 20,000 g, 30 min, and 4°C. The supernatant was identified as cell-free crude extract and tested for a reduction reaction of the CF₃ ketone. The enzyme activity of the respective crude extracts was measured using the following batch: 200 µl crude extract, 1 mM NAD⁺, 1 mM NADP⁺, 1 mM MgCl₂, 100 mM β-D-glucose, 10 mM rac CF₃ ketone (dissolved in methanol), 20 µl glucose dehydrogenase (GDH) (crude extract, 250 U/ml) from *Bacillus subtilis* and 50 mM KPi buffer pH 7.5 in 1 ml total volume. The batches were incubated in a thermal mixer at 900 rpm and 30°C for 20 hours.

### b) gas chromatographic analysis

The reduction of the racemic CF₃ ketone was measured by means of gas chromatography. For this, 200 µl of ethylacetate was added to 100 ul of the samples, the mixture was vortexed for 1 minute and afterwards phases were separated by centrifugation at 14,000 rpm for 5 min. The organic phase was used for gas chromatographic analysis. The chromatography was carried out on a GC-17A gas chromatograph (Shimadzu, Germany) using a β-cyclodextrin column (CP-Chirasil-Dex CB column, 25 m x 0.25 mm, Varian, Germany). Compounds were analyzed using the following temperature steps: 60°C (5 min); 30°C/min to 135°C; 5°C/min to 185°C, 30°C/min to 195°C (0 min) and the corresponding retention times were monitored: 11.74 min for the (*S*)-enantiomer of the CF₃ ketone, 11.94 min for the (R)-enantiomer of the CF₃ ketone, 15.90 min for the 1,3-diol of the (*S*)-CF₃ ketone.

### c) Cultivation, lysis and purification of Pichia pastoris GS 115

Cells of the strain *Pichia pastoris* GS 115 were cultivated in yeast extract peptone dextrose (YPD) medium at 25°C for three days and afterwards harvested by centrifugation. *Pichia* cells were resuspended in KPi buffer (50 mM, pH 7) to yield a 25% cell suspension. The cell suspension was disrupted using a French Press (Glen Mills Inc, Clifton) for three times at 900 psi under cooling. Centrifugation at 20,000 g, for 45 min, and 4°C yielded a cude extract which was used for ammonium sulfate precipitation. For this, 22.5 g ammonium sulfate were added to 69 ml of the crude extract to yield a 55% saturation, and incubated on ice for 1 hour under stirring. Afterwards a part of the proteins of the *Pichia* crude extract were centrifuged off. The resulting supernatant was subjected to a second ammonium sulfate precipitation using 15.8 g ammonium sulfate and 69 ml of crude extract to yield a 90% saturation and incubated. After centrifugation the supernatant was discarded and the sediment was resuspended in buffer (100 mM Kpi buffer, 1.7 M ammonium sulfate, pH 7.2). The solution was transferred to a Phenyl Sepharose 6 Fast Flow column (GE Healthcare, Amersham, UK) which was equilibrated using 5 column volumes (CV). The proteins were eluted using Kpi buffer (50 mM, pH 7) and a gradient of 1.7 M to 0 M ammonium sulfate. The enzyme activity was determined and the active fractions were combined and rebuffered using an ultra filtration membrane (Mr 10,000 Da) and 50 mM tris HCl buffer pH 9. After concentration the protein suspension was transferred to a Q Sepharose 6 Fast Flow column (GE Healthcare, Amersham, UK) equilibrated with 5 column volumes (CV) tris HCl buffer (50 mM, pH 9). The same buffer was run as wash buffer through the column, and the proteins were eluted by means of a linear NaCl gradient of 0 M to 1 M NaCl in tris HCl buffer (50 mM, pH 9) and 1 M NaCl. The active fractions were concentrated, rebuffered to Kpi buffer (50 mM + 300 mM NaCl, pH 7.2) and transferred to a Superdex 75column (GE Healthcare, Amersham, UK) equilibrated with Kpi buffer (50 mM + 300 mM NaCl, pH 7.2). The resulting fractions were tested for enzyme activity in reducing the CF₃ ketone using gas chromatographic analysis. After each purification step the protein concentration was determined and samples were analysed using SDS-PAGE.

Figure 2 shows the SDS-PAGE of the fractions containing *Pichia pastoris* alcohol dehydrogenase (PPADH) after the respective purification steps. Lane 1 shows the crude extract (10 µg) after ammonium sulfate precipitation, lane 2 the sample after hydrophobic interaction chromatography, lane 3 the eluate after ion-exchange chromatography (10 µg, precipitated using trichloro acetic acid (TCA)). Lane M shows the protein ladder (Prestained Protein Ladder, Thermo Scientific). As can be taken from the Figure 2, two protein bands of about 36 kDa and 39 kDa intensified in each purification stage. These two protein bands were excised, digested with trypsin, and the resulting peptides were analysed by MALDI-TOF mass spectrometry.

Two protein bands could be identified as being 1) "glyceraldehyde-3-phosphate dehydrogenase" (Uniprot-ID: C4R0P1), 35.6 kDa and 2) "putative dihydrokaempferol 4-reductase" (Uniprot-ID: C4R4L0), 39.2 kDa from *Pichia pastoris* GS115. "Glyceraldehyde-3-phosphate dehydrogenase" is an ubiquitous enzyme, which is existant in almost every organism. Thus, the gene of the "putative dihydrokaempferol 4-reductase" (Uniprot-ID: C4R4L0) which also is denoted PPADH from *Pichia pastoris* GS 115 was cloned into the expression vector pET28-a(+). For this, the PPADH gene was amplified by means of polymerase chain reaction (PCR) and provided with an *Ndel* cleavage site and a *Xho*I cleavage site using forward primer 5'-GCGCTTCCATATGGTTTCTAAGG-3' (SEQ ID NO:4) and reverse primer 5' CGGCTCGAGATTATTTATTAGCACGC 3'(SEQ ID NO:5). After restriction of the pET-28a(+) vector with the restriction enzymes *Ndel* and *Xho*I, the PPADH gene was cloned into the vector. The resulting vector was referred to as pET28a-PPADH. Competent *E. coli* BL21(DE3) cells were transformed with the vector for expression. After induction with IPTG and cultivation, the cells were harvested, resuspended in tris HCl buffer to yield a 25% cell suspension and disrupted by sonification. Centrifugation yielded the cell-free crude extract which was screened for its activity in reducing CF₃ in the assay as described in example 2a) and verified by means of gas chromatography as decribed in example 2b). Surprisingly, a distinct reduction of the (*S*)-enantiomer of the racemic CF₃ ketone to the corresponding 1,3-diol was measured by the crude extract of the *E. coli* BL21(DE3) containing the recombinant PPADH.

### Example 3: Heterologous expression of PPADH in E. coli BL21(DE3) and purification

### a) Effect of temperature on the expression

For the expression of PPADH *E. coli* BL21(DE3) cells were transformed with the vector pET28a-PPADH and cultivated in kanamycin-containing LB or TB medium. The pET-28a(+) vector carries an N-terminal 6xHis tag in addition to an optional C-terminal His tag sequence. Cells were grown to an optical density (at 600 nm) of 0.5 to 0.7 at 37°C, and when arriving at this optical density the expression of PPADH was induced using 0.5 mM isopropyl β-D-1-thiogalactopyranoside (IPTG) solution. Subsequently, the cell suspension was incubated at 20 hours at 20°C, 24°C, 30°C and 37°C in order to achieve the optimal PPADH yield. After cultivation the cells were harvested via centrifugation (18,000 g, 30 min, 4 °C). Cells were resuspended in 10 mM tris HCl buffer (pH 7) to yield a 25% cell suspension and disrupted by sonification under cooling. Three cycles of 2 min of 25% power output followed by 1 min cooling were used. After repeated centrifugation a cell-free crude extracts were obtained.

The reductive enzymatic activity of the crude extracts was measured using a photometric activity assay. The reduction was determined using 10 mM racemic CF₃ ketone in tris HCl buffer (100 mM, pH 7), 0.25 mM NADPH and 10 µl enzyme (crude extract). The reaction was performed in 1 cm-cuvettes. The compounds were mixed in a cuvette, the cuvette was placed in the photometer and data recording was started. The enzyme solution was added only immediately before the start of the measurement. The activities of the enzyme were determined by spectrophotometric detection of the reaction of NADH to give NAD⁺ after certain time intervals. Spectrophotometric measurement was carried out at a temperature of 25°C, a wavelength of 340 nm and over a time of 1 minute.

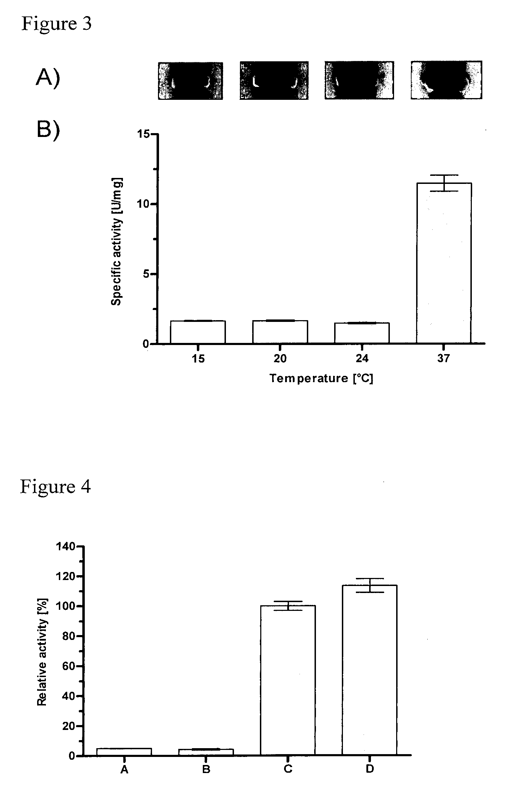

Figure 3A) shows the influence of the expression temperature on soluble expression of PPADH and Figure 3B) shows the specific activity of PPADH in *E. coli* BL21(DE3) cells at 20°C, 24°C, 30°C and 37°C. Activity measurements were run in triplicate, and the standard deviation is shown in the diagramm. As can be taken from the Figure 3, expression at 37°C yielded a higher specific activity compared to the lower expression temperatures. It is assumed that a better over expression was reached at 37°C.

### b) Effect of the His tag on enzyme activity

The effect of N-terminal and C-terminal His tags on the enzymatic activity of PPADH were determined using the following variants: [A] with-out His tag, [B] with C-terminal His tag, [C] with N-terminal His tag and [D] with N- and C-terminal His tag. These gene variants were overexpressed in *E. coli* BL21(DE3) at 37°C under equal conditions as described in example 3a). The crude extract of each of the variants was isolated and the activity regarding the reduction of benzaldehyde was tested. The enzymatic activity of the extracts were determined using 20 mM benzaldehyde in tris HCl buffer (100 mM, pH 7), 0.20 mM NADPH and 10 µl enzyme (crude extract). The reaction was performed in 1 cm-cuvettes. The compounds were mixed in the cuvette used for the experiment, which was placed in the photometer and data recording was started. The enzyme solution was added only immediately before the start of the measurement. The activity of the enzyme was determined by photometric detection of the reaction of NADPH after certain time intervals. Spectrophotometric measurement was carried out at a temperature of 25°C, a wavelength of 340 nm and over a time interval of 1 min.

Figure 4 shows the influence of an N-terminal and C-terminal His tag on the enzymatic activity of PPADH in the crude extracts. The relative activity of PPADH is shown for the following variants: [A] with-out His tag, [B] with C-terminal His tag, [C] with N-terminal His tag and [D] with N- and C-terminal His tag. 100% relative activity corresponds to the specific activity of 1.22 U/mg in the crude extract. Samples were run in triplicate, and the respective standard deviation is shown in the diagramm. As it can be seen in Figure 4, that an N-terminal or C-terminal His tag does not have a negative effect on the enzymatic activity of PPADH compared to the wild-type without modification. Compared to the native enzyme the variant carrying an N-terminal His tag however exhibits a much higher enzyme activity. A C-terminal His tag on the other hand appears to exert no influence on enzyme activity, as this variant shows an activity that as comparable to that of the wild-type without modification. The variant comprising an N- and a C-terminal His tag showed increased enzyme activity as does the variant carrying the N-terminal His tag. It thus could be shown that fusion of PPADH with an N-terminal His tag leads to an increase in enzyme activity of about 95%.

### c) Purification of PPADH via IMAC

The N-terminal His tag allows for a purification of PPADH enzyme by means of Immobilized Metal Affinity Chromatography (IMAC) using a column with a nickel-charged nitrilotriacetic acid (Ni-NTA) matrix. Firstly, the PPADH gene was overexpressed in *E. coli* BL21 (DE3) at 37°C as described in example 3a). The obtained bacterial culture was harvested by centrifugation (18,000 g, 30 min, 4 °C). Cells were resuspended in 100 mM tris HCl buffer (pH 7) to yield a 25% cell suspension and disrupted by sonification under cooling in ice. Three cycles of 2 min of 25% power output followed by 1 min cooling were used. After centrifugation (18,000 g, 30 min, 4 °C) the cell-free crude extract was used for Ni-NTA protein purification (Ni-NTA (mclab, Netherlands). The column was equilibrated using lysis buffer (50 mM NaH₂PO₄, 300 mM NaCl, 10 mM imidazole, pH 8). Subsequently, the column was loaded with protein extract and unbound protein was removed using wash buffer (50 mM NaH₂PO₄, 300 mM NaCl, 20 mM imidazole, pH 8). PPADH was eluted using 250 mM imidazole in elution buffer (50 mM NaH₂PO₄, 300 mM NaCl, 250 mM imidazole, pH 8), desalinated using a PD-10 column (Sephadex G 25 (GE Healthcare), and rebuffered in 100 mM tris buffer pH 7. The purified enzyme was stored at 4°C until use. Table 1 summarizes the results of the purification.

**Tabelle 1: purification of recombinant PPADH from E. coli BL21(DE3) cells**

| Purification step | Total activity [U] | specific activity [U/mg] | Purification factor | yield [%] |
|---|---|---|---|---|
| Crude extract | 117 | 3.6 | 1 | 100 |
| purified PPADH | 78 | 37.3 | 10 | 66.9 |

The activity of PPADH in the crude extract and of the purified enzyme were determined by photometry as described in example 3b). Ni-NTA purification resulted in a yield (recovery) of about 67%. The specific activity of PPADH was 37.3 U/mg after purification, which corresponds to a 10-fold increase in specific activity compared to the crude extract.

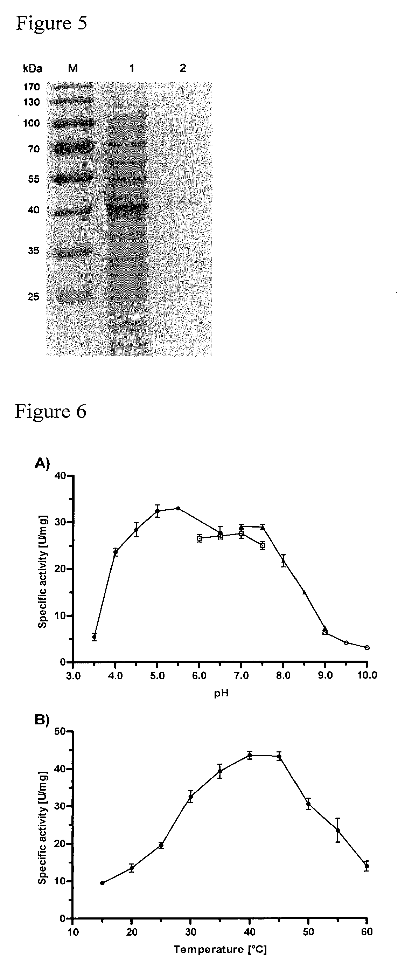

Figure 5 shows a picture of the SDS agarosegel of the purified PPADH carrying an N-terminal His tag from *Pichia pastoris.* Lane 1 shows the crude extract, lane 2 the purified ppADH. Each lane was loaded with 10 µg protein. M shows the molecular ladder (5 µl). it can be seen in the Figure 5 that the purification was successful and yielded 95% of the designated protein via only one purification step.

### Example 4: Biochemical characterisation of the PPADH from Pichia pastoris

### a) Temperature dependence, pH dependence and storage stability

In order to determine the activity of the recombinant PPADH as a function of pH, the enzyme activity was determined spectrophotometrically as described under example 3b) but using 10 mM ethyl-4-chloroacetoacetate instead of benzaldehyde as substrate. The reduction of ethyl-4-chloroacetoacetate was carried out at 25°C for one minute, with the oxidation of NADPH being monitored via the decrease in extinction at 340 nm. The measurement was carried out in the following buffers with overlapping pH ranges: citrate-phosphate buffer for the range pH 4.0 to 6.0, KPi buffer for the range pH 6.0 to 8.0, tris HCl buffer for the range pH 7.0 to 9.0 and natrium-carbonate buffer for the range pH 9.0 to 10.0. Samples were run in triplicate, and the respectve standard deviation is shown in the diagramm. Figure 6A) shows the effect of pH on the specific activity of PPADH in the reduction of ethyl-4-chloroacetoacetate. Figure 6A) shows that PPADH exhibits high activity in a range of pH 4.0 to 8.0. The optimum of PPADH enzymatic activity in the reduction of the carbonyl compound was at about pH 5.5 in citrate-phosphate buffer. The broad pH range of high activity of PPADH is particularly advantageous for commercial use as catalyst.

In order to determine the activity of the recombinant PPADH as a function of temperature, the enzyme activity was determined spectrophotometrically as described under example 3b) but using 10 mM ethyl-4-chloroacetoacetate in 0.1 M tris-HCl buffer (pH 7) instead of benzaldehyde as substrate. The reduction of ethyl-4-chloroacetoacetate was carried out at temperatures in a range from 15°C to 60°C for one minute. Figure 6B) shows the effect of temperature on the specific activity of PPADH in the reduction of ethyl-4-chloroacetoacetate. Figure 6B) shows that PPADH exhibits high activity in catalysing the reduction at a temperature in a range from 20°C to 60°C, particularly in the range from 30°C to 45°C.

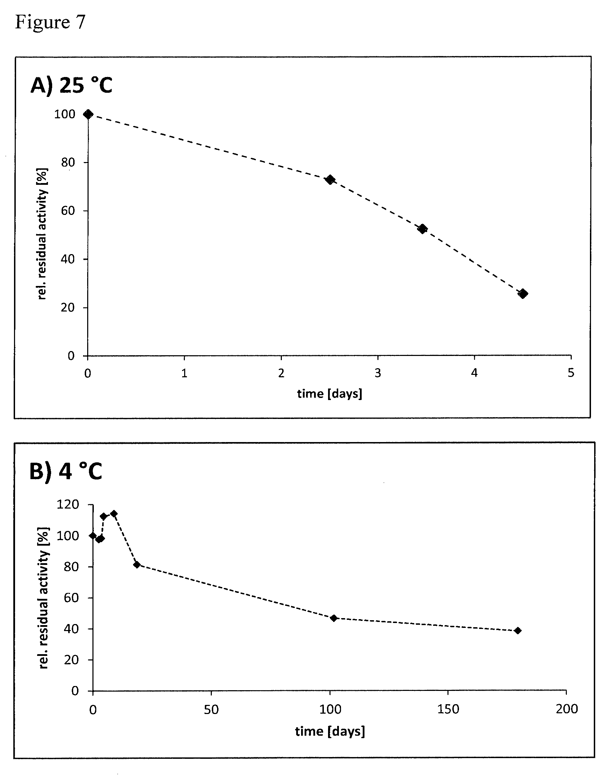

In order to determine the storage stability of the recombinant PPADH, enzyme samples were at 4°C and 25°C. Samples were taken out at various times, and the residual activity was measured spectrophotometrically as described under example 3b) using benzaldehyde as substrate and NADPH. Figure 7A) shows the residual activity of PPADH after storage for 4.5 days at 25°C, and Figure 7B) the residual activity of PPADH after storage for about 180 days at 4°C. Initial activity at t=0 was set to 100%. The Figure 7A) shows that activity of PPADH decreased at 25°C in a few days, and PPADH exhibited a half-life period at 25°C of about 2.5 days. At 4°C PPADH however showed good stability and preserved residual activity over a long time. PPADH exhibited a half-life period at 4°C of about 114 days. This shows that recombinat PPADH can be stored at 4°C with preserving ca. 50% residual activity over a relatively long time.

### b) Determination of substrate spectrum of the recombinant alcohol dehydrogenase PPADH

In a first step, crude extract containing recombinant alcohol dehydrogenase from *Pichia pastoris* via cell disruption of *E. coli* BL21(DE3) overexpressing in vector pET28a-PPADH carrying an N-terminal 6xHis tag. Cells were resuspended in 10 mM tris HCl buffer (pH 7) to yield a 25% cell suspension and were disrupted by sonification under cooling. Three cycles of 2 min of 25% power output followed by 1 min cooling were used. After repeated centrifugation a cell-free crude extract was obtained. Afterwards the PPADH was purified as described in example 3c).

Determination of the enzymatic activity of the purified PPADH can be determined via a oxidation or reduction reaction. For determination of the reduction the following compounds were used: tris HCl buffer (100 mM, pH 7), 0.2 mM NADPH, 10 mM substrate selected from the aldehydes and ketones as given in Table 2 and 10 µl enzyme (purified PPADH). For determination of the oxidation the following compounds were used: tris HCl buffer (100 mM, pH 9), 0.2 mM NADP⁺, 10 mM substrate selected from the alcohols as given in Table 3 and 10 µl enzyme (purified PPADH). The reaction was performed in 1 cm-cuvettes. The compounds were mixed in the cuvette used for the experiment, which was placed in the photometer and data recording was started. The enzyme solution was added only immediately before the start of the measurement. The activity of the enzyme was determined by photometric detection of the concentration of NADPH after a certain time interval. Measurement was carried out at a temperature of 25°C, a wavelength of 340 nm and over a time interval of 1 min.

**Table 2: enzymatic activity of PPADH regarding the reduction of different substrates determined by photometry**

| sample | substrate | specific activity [U/mg] | relative activity [%] |
|---|---|---|---|
| 1 | 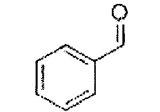 | 38.6 | 100.0 |
| 2 | 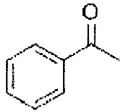 | 0.06 | 0.2 |
| 3 | 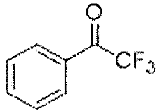 | 0.10 | 0.3 |
| 4* | 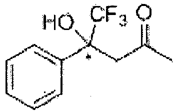 | 37.5 | 97.2 |
| 5 | 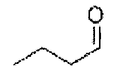 | 68.0 | 176.2 |
| 6 | 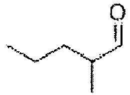 | 96.1 | 248.9 |
| 7 | 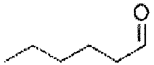 | 28.5 | 73.9 |
| 8 | 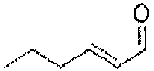 | 37.5 | 97.2 |
| 9 | 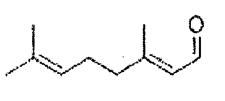 | 17.8 | 46.1 |
| 10 | 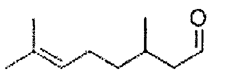 | 27.8 | 71.9 |
| 11 | 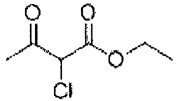 | 5.7 | 14.7 |
| 12 | 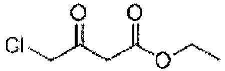 | 38.0 | 98.3 |
| 13 | 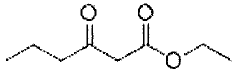 | 0.5 | 1.3 |
| 14 | 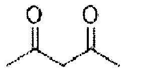 | 3.3 | 8.5 |

| | | | |
|---|---|---|---|
| * 20 mM CF₃-ketone (rac) was used in the assay. | | | |

The specific activity of 38.6 U/mg of purified PPADH in reducing benzaldehyde was set to 100% relative activity. As can be seen in the Table 2, PPADH showed good reductive activities.

In further examples benzylacetone, cyclohexen-1-on, fenchon, 3,3,5-trimethylcyclohexanone, propiophenone, (-)-carvone, 2-hexanone, 2-nonanone, 2-octanone, (+)-pulegon, (-)-menthone, 4-methyl-2-pentanone, 4-methoxy-4-metyhl-2-pentanone, dihydro-4,4-dimethyl-2,3-furandion, 4-hydoxy-3-hexanone, acetone and ethylacetate were tested, but PPADH showed no detectable or low activity (<0.5 U/mg) towards these substrates in the photometric assay.

**Table 3: enzymatic activity of purified PPADH regarding the oxidation of different alcohols determined by photometry**

| sample | substrate | specific activity [U/mg] | relative activity [%] |
|---|---|---|---|
| 15 | 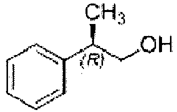 | 1.06 | 16.0 |
| 16 | 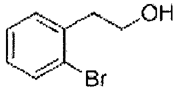 | 1.94 | 29.3 |
| 17 | 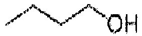 | 1.11 | 16.7 |
| 18 | 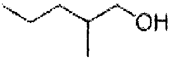 | 2.04 | 30.9 |
| 19 | 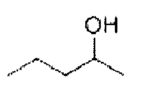 | 2.53 | 38.3 |
| 20 | 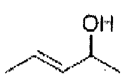 | 1.29 | 19.5 |
| 21 | 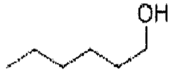 | 1.26 | 19.1 |
| 22 | 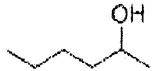 | 3.62 | 54.9 |
| 23 | 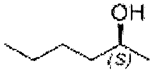 | 6.61 | 100.0 |
| 24 | 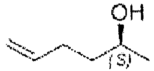 | 3.79 | 57.4 |
| 25 | 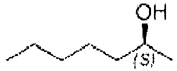 | 2.09 | 31.6 |
| 26 | 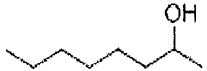 | 0.57 | 8.7 |
| 27 | 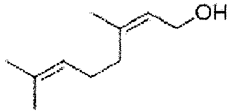 | 2.16 | 32.7 |
| 28 | 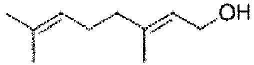 | 1.19 | 18.0 |

A specific activity of 6.6 U/mg of purified PPADH in oxidising a substrate was set to 100% relative activity. As can be seen in the Table 3, PPADH showed good oxidative activities.

In further examples (R)-(-)-5-hexen-2-ol, 2-phenyl-1-propanol, benzylalcohol, (S)-(+)-2-amino-1-butanol, (R)-(-)-2-octanol, 1,2-butanediol, 1,2-propanediol, octen-3-ol, cyclohexanol, 2,3-butanediol, 1-(4-chlorophenyl)ethanol, (S)-(+)-2-butanol, 1-penten-3-ol, 2-cyclohexen-1-ol, and (R)-(+)-1-phenylethanol were tested, but PPADH showed no detectable or minor activity (<0.5 U/mg) towards these substrates in the photometric assay.

### c) Determination of kinetic parameters of the recombinant alcohol dehydrogenase PPADH

In order to determine the kinetic parameters of the recombinant PPADH, purified enzyme was manufactured as described in example 4b). Afterwards the described test for reductive activity was used. All parameters such as 100 mM tris HCl buffer (pH 7), 0.2 mM NADPH, and 10 µl enzyme (purified PPADH) were kept constant, except that concentration of substrate and concentration of co-factor were varied. The kinetic parameters were calculated using the software GraphPad Prism5 based on a non-linear ,,fitting"-algorithm. The kinetic parameters are summarized in the following Table 4.

**Table 4: kinetic parameters of purified PPADH regarding benzaldehyde, racemic CF₃-ketone as substrates and the co-factor NADPH determined by photometry**

| Substrate | K_{M} [µM] | Vₘₐₓ [U/mg] | kcat [s⁻¹] | kcat/K_{M} [s⁻¹mM⁻¹] |
|---|---|---|---|---|
| benzaldehyde | 3034.0 | 98.5 | 25.1 | 8.3 |
| CF₃-ketone (rac) | 1797.0* | 46.3 | 6.4 | 3.6* |
| NADPH | 48.3 | 94.2 | 19.5 | 403.9 |

| | | | | |
|---|---|---|---|---|
| * PPADH is enantioselective, so only the (S)-ketone is reacted. Since the racemic CF₃-ketone used as substrate comprises only 50% of (*S*)-enantiomer, the K_{M}-value should be 898.5 µM and the kcat/K_{M}- value should be 7.1 s⁻¹mM⁻¹. | | | | |

Table 4 shows that the PPADH exhibits higher substrate affinity towards the CF₃ ketone compared to benzaldehyde as substrate (K_{M} for CF₃ < K_{M} for benzaldehyde). In conclusion the PPADH shows good kinetic parameters for the reduction of benzaldehyde and CF₃ ketone, whereby using NADPH as co-factor.

### Example 5: Use of the recombinant alcohol dehydrogenase PPADH for producing diastereomerically pure (S,S)-1,3-diol using PPADH as crude extract

The following scheme 4 shows the enzymatic kinetic resolution of racemic CF₃ ketone by reduction of only the (*S*)-enantiomer to the corresponding 1,3-diol using PPADH and glucose dehydrogenase for co-factor regeneration.

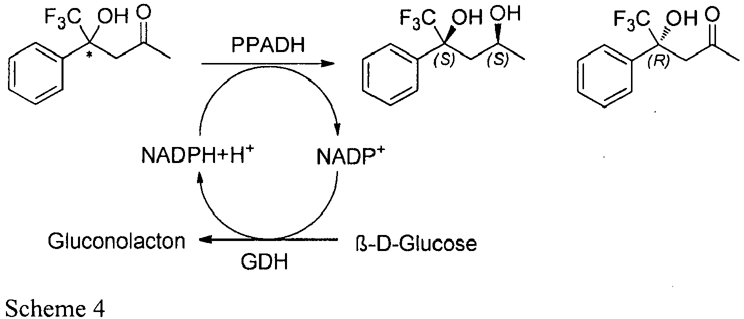

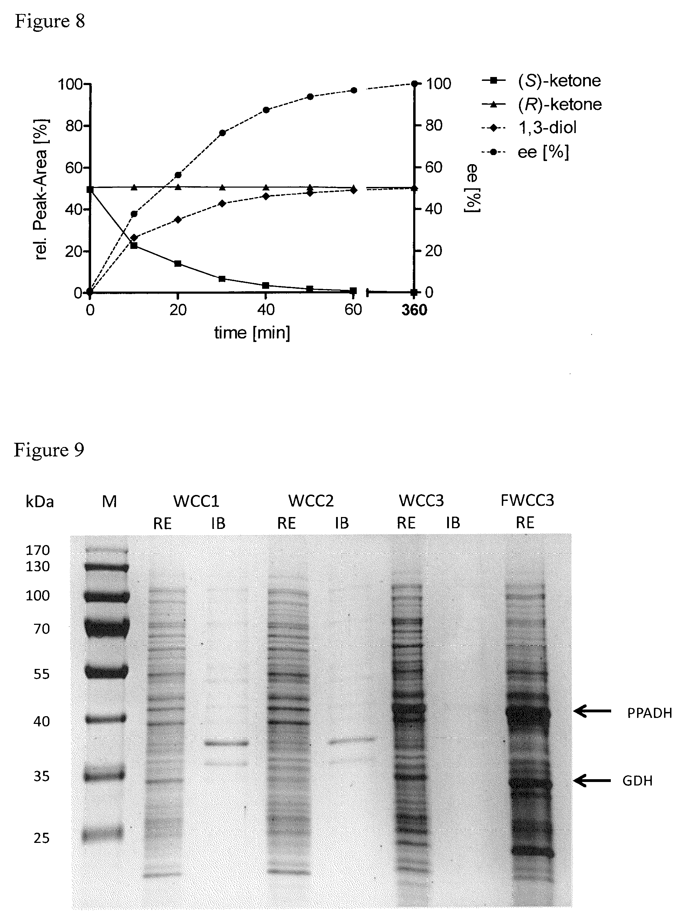

The enzymatic reaction was performed using a crude extract of PPADH as described above. The co-factor was regenerated with glucose dehydrogenase (GDH). For this, 20 mM racemic CF₃ ketone, 0.24 U/ml PPADH, 100 mM glucose, 2 U/ml GDH and 1 mM NADP in 50 mM triethanolamine hydrochloride (TEA) buffer pH 7 were used. The reaction was performed at a temperature of 30°C and the enzymatic reduction of the (S)-enantiomer of the racemic CF₃ ketone was observed for 6 hours. Samples were taken after 0, 10, 20, 30, 40, 50, 60 minutes and 6 hours. The Figure 8 shows the result of the reaction given as the relative peak area of the (S)-ketone, the (R)-ketone and the diol versus time. As can be taken from the Figure 8, PPADH enantioselectively reduced the (S)-ketone. The amount of the (S)-enantiomer decreased over time, and was reduced to the corresponding diol. The amount of the (R)-enantiomer remained constant, which means that it was enriched in the mixture. After one hour the enantiomeric excess (ee), which reflects the degree to which a sample contains one enantiomer in greater amount than the other, was determined to be 96.9%. This shows that PPADH is an enzyme potentially usable for the kinetic resolution of the racemic CF₃ ketone and for the synthesis of enantio-pure and diastereomerically pure 1,3-diol.

### Example 6: Selected biotransformations of the alcohol dehydrogenase PPADH from Pichia pastoris with co-factor regeneration system

The reduction of selected acetophenone derivatives by purified PPADH was tested in a co-factor regenerating system using a crude extract of glucose dehydrogenase (GDH). The following assay was used: substrate dissolved in 50 µl methanol, 10 µl NADP⁺ (100 mM), 100 µl purified PPADH, 40 µl glucose (2.5 M), 50 µl GDH (crude extract) and 750 µl tris HCl buffer (0.1 M, pH 7) in a total volume 1 ml. The reaction products were separated by gas chromatography. The activity of the purified PPADH referred to benzaldehyde was determined to be 22.4 U/ml (37.3 U/mg). The activity of the crude extract of glucose dehydrogenase referred to D-glucose was determined to be 237.9 U/ml (8.9 U/mg).

The following Table 5 shows the levels of turnover for the acetophenone derivatives after 2 hours reaction time related to the alcohol formed. The levels of turnover are indicated as follows: -=0-1%, +=1-5%, ++=5-20%, +++=20-55%.

**Table 5: levels of turnover for acetophenone derivatives**

| No. | structure | name | concentration [mM] | turnover | level of turnover |
|---|---|---|---|---|---|
| 1 | 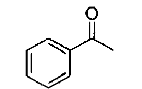 | acetophenone | 22.5 | 7.8 % (S) | ++ |
| | *ortho-acetophenone derivates* | | | | |
| 1 | 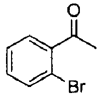 | 2'-bromoacetophenone | 11.6 | <5 % | + |
| 2 | 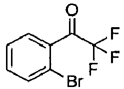 | 2'-Bromo-2,2,2-trifluoroacetophenone | 8,3 | <5 % | + |
| 3 | 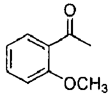 | 2'-Methoxyacetophenone | 13.3 | <5 % | + |
| | *para- acetophenone derivate* | | | | |
| 4 | 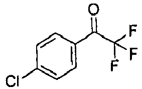 | 4'-Chloro-2,2,2-trifluoro acetophenone | 12.9 | 12.4 % ee= 63.8 % | ++ |
| | *substituted acetophenone derivates* | | | | |
| 5 | 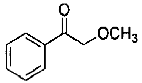 | 2-Methoxyacetophenone | 17.3 | <1 % | - |
| 6 | 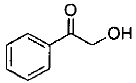 | 2-Hydroxyacetophenone | 19.1 | <5 % | + |
| 7 | 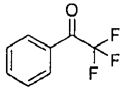 | 2,2,2-Trifluoroacetophenone | 15.5 | 24.2 % ee= 29.5 % | +++ |

Table 5 shows that acetophenone derivatives are not good substrates for the PPADH in comparison to aldehydes or the CF₃-ketone. It can be seen that the PPADH shows higher conversions towards halogenated acetophenone derivatives with exception of ortho-substituted acetophenone derivatives. Additionally, the PPADH reduces acetophenone enantiospecifically to (S)-phenylethanol, surprisingly the reduction of 2,2,2-trifluoroacetophenone and 4'chloro-2,2,2-trifluoroacetophenone lead not to the synthesis of enantiomerically pure alcohols assuming that halogens present in the substrate influence the enantioselectivity of PPADH.

In a further test series, the reduction of CF₃-ketone derivatives by purified PPADH was tested with co-factor regenerating system using glucose dehydrogenase (GDH). The following assay was used: substrate dissolved in 50 µl methanol, 10 µl NADP⁺ (100 mM), 100 µl purified PPADH, 40 µl glucose (2.5 M), 50 µl GDH (crude extract) and 750 µl tris HCl buffer (0.1 M, pH 7) in a total volume 1 ml. The activity of the purified PPADH referred to benzaldehyde was determined to 9.4 U/ml (47.0 U/mg). The activity of the crude extract of glucose dehydrogenase referred to D-glucose was determined to 237.9 U/ml (8.9 U/mg).

The following Table 6 shows the levels of turnover for the CF₃-ketone derivatives after 2 hours reaction time for the alcohol formed related to the reduction of (S)-ketone. The levels of turnover are indicated as follows: - = 0-1%, + =1-5%, ++ = 5-20 %, +++ =20-55 %, ++++ = 50 - 75 %, +++++ = 75 - 100 %, quant=quantitative (100% turnover).

**Table 6: levels of turnover for CF₃-ketone derivatives**

| No. | strukture | name | concentration [mM] | turnover | level of turnover |
|---|---|---|---|---|---|
| 1 | 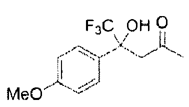 | Nod365 F6 S enriched (ca. 70 %) | 9.9 | quant. | +++++ |
| 2 | 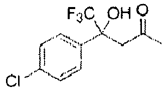 | Nod362 rac | 8.3 | quant. | +++++ |
| 3 | 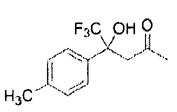 | Nod364 F3-Enon S enriched (94.8%) | 10.2 | quant. | +++++ |
| 4 | 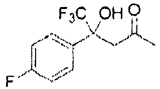 | Nod363 F4 S enriched (73.4%) | 9.2 | quant. | +++++ |
| 5 | 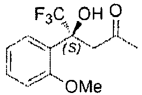 | Nod-3d S enriched (93.3 %) | 9.9 | 75.8% | +++++ |
| 6 | 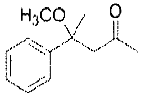 | NOD-VI-405 rac. | 12.5 | 20.3% | +++ |
| 7 | 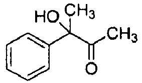 | CST060 rac? | 15.2 | 18.9% | ++ |
| 8 | 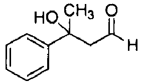 | CST070 rac? | 19.5 | 30.4% ee= 13.3 % | +++ |
| 9 | 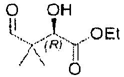 | MHA369 R enriched (76 %) | 15.5 | 96.9% ee= 62.9 % | +++++ |

Table 6 shows that the PPADH accepts also derivatives of the CF₃ ketone as can be taken from entry No. 1-5, whereby para-substituted CF₃ ketones are preferred over ortho-substituted CF₃ ketones as can be taken from a comparison of entry 1-4 with entry 5. Other substrates showing similar structures were also accepted as substrates but with lower conversion rates as can be taken from entry 6-8.

### Example 7: Synthesis of diastereomerically pure (S,S)-1,3-diol using a whole-cell catalyst

### a) Construction of a recombinant E. coli strain for whole-cell catalysis

The following whole-cell constructs (WCCs) were produced for the reduction of the CF₃-ketone:
Construct 1 (WCC1): The plasmid pETDuet-1 (Amp^{R}) carrying two multiple cloning sites (MCS) was used for the construct 1. The *ppadh* gene was cloned into the first multiple cloning site (MCS1) via *Nco*I & *Not*I cleavage sites. The *gdh* gene was cloned into the second multiple cloning site (MCS2) via *Nde*I *& Xho*I cleavage sites. Thus, MCS 1 provides for the PPADH protein and MCS2 for GDH protein.
Construct 2 (WCC2): The plasmid pETDuet-1 (Amp^{R}) carrying two multiple cloning sites (MCS) was used. For the construct 2, the *gdh* gene was cloned into MCS 1 via *Bsa*I & *Not*I cleavage sites, while the *ppadh* gene was cloned into the second multiple cloning site (MCS2) via *Nde*I & *Xho*I cleavage sites. Thus, in construct 2 MCS 1 provides for the GDH protein and MCS2 for the PPADH protein.
Construct 3 (WCC3): The plasmid pACYC-Duet-1 (Cm^{R}) was restricted using *Nco*I & *Xho*I, for cloning the *gdh* gene via the same *Nco*I & *Xho*I restriction sites. This restriction in parallel removes the MSC2 for preserving only the necessary components for overexpression. Bothe plasmids can be transformed in *E. coli* BL21 (DE3) and the genes be expressed, since this plasmid contains another "ori" than the vector pET28a. The resulting vectors are referred to as pET28a-PPADH (Km^{R}) and pACYC-GDH (Cm^{R}).

Successful preparation of the three constructs was confirmed by sequencing. The three whole-cell constructs afterwards were transformed in *E. coli* BL21(DE3). Bacterial cultures in 500 ml LB medium were inoculated with a starting OD of 0.05 at 37°C and upon arriving at an OD of 0.5 to 0.7 the expression was induced using 0.5 mM isopropyl β-D-1-thiogalactopyranoside (IPTG). The cell cultures were harvested after an incubation of about 20 hours. For each whole-cell construct a 25% cell suspension was prepared which was disrupted by sonification.

The thus obtained cell-free crude extracts were determined for activity of alcohol dehydrogenase (ADH) and glucose dehydrogenase (GDH). The following reactants were used for the reduction of benzaldehyde by PPADH at 25°C: 980 µl of substrate solution containing 20 mM benzadehyde in 0.1 M tris HCl buffer of pH 7, 10 µl NADPH (20 mM) and 10 µl crude extract in a total volume of 1 ml. The following reactants were used for the reduction of the racemic CF₃-ketone by PPADH at 25°C: 980 µl of substrate solution containing 20 mM CF₃-ketone in 0.1 M tris HCl buffer of pH 7, 10 µl NADPH (20 mM) and 10 µl crude extract in a total volume of 1 ml. The following reactants were used for the oxidation of D-glucose by GDH at 25°C: 980 µl of substrate solution containing 100 mM D-glucose in 0.1 M tris HCl buffer of pH 7, 10 µl NADP⁺ (20 mM) and 10 µl crude extract in a total volume of 1 ml.

In both tests for the reduction of benzaldehyde and CF₃-ketone, respectively, the substrate solution and the co-factor (total volume 990 µl) were incubated in a micro cuvette for 5 minutes at 25°C. Afterwards the enzyme was added and the solution stirred. The decrease or increase of the co-factor was monitored photometrically for 1 minute at 340 nm. This allows to draw conclusions on the activity of the enzyme, as the co-factor will be reduced in a oxidation reaction and oxidized in a reductive reaction. The following Tables 7 and 8 summarize the measured enzyme activities in the crude extracts of the whole-cell constructs.

**Table 7: Comparison of the activities of GDH and PPADH in the whole-cell crude extracts regarding the reduction of benzaldehyde**

| whole-cell constructs (WCC) | activity GDH2 [U/ml] | activity GDH2 [U/mg] | activity PPADH [U/ml] | activity PPADH [U/mg] | PPADH/GDH2 [%] |
|---|---|---|---|---|---|
| WCC1: pETDuet-PPADH-GDH2 | 40.66 | 2.98 | 7.46 | 0.55 | 18 |
| WCC2: pETDuet-GDH2-PPADH | 8.84 | 0.65 | 5.88 | 0.44 | 67 |
| WCC3: pACYC-GDH2 + pET28a-PPADH | 28.01 | 1.90 | 46.73 | 3.18 | 167 |
| fermented WCC3 | 62.94 | 4.57 | 104.25 | 7.56 | 166 |

**Table 8: Photometric activity of PPADH in the whole-cell crude extracts regarding the reduction of CF₃-ketone**

| whole-cell constructs (WCC) | activity PPADH [U/ml] | activity PPADH [U/mg] | PPADH/GDH2 [%] |
|---|---|---|---|
| WCC 1: pETDuet-PPADH-GDH | 7.15 | 0.52 | 18 |
| WCC2: pETDuet-GDH-PPADH | 4.79 | 0.35 | 54 |
| WCC3: pACYC-GDH+ pET28a-PPADH | 44.68 | 3.04 | 159 |
| fermented WCC3 | 100.60 | 7.30 | 160 |

As can be taken from the Tables 7 and 8, the enzymes PPADH and GDH both were expressed in an active form. The activity of PPADH however was lower than the activity of GDH. Regarding the construct WCC2 the activity of PPADH was significantly lower compared to the constructs WCC1 and WCC3. This was confirmed by the SDS page shown in the Figure 9. Figure 9 shows the SDS page of the crude extracts. Lane 1 shows construct WCC1, lane 2 shows construct WCC2, lane 3 shows construct WCC3. RE shows the crude extracts, IB inclusion bodies. All lanes were loaded with 10 µg protein. M shows a prestained marker (Thermo Scientific). As can be seen in Figure 9, specific protein bands were seen only for the crude extracts of whole cells for constructs WCC1 and WCC3.

### b) Reaction of benzaldehydes with the whole-cell catalysts

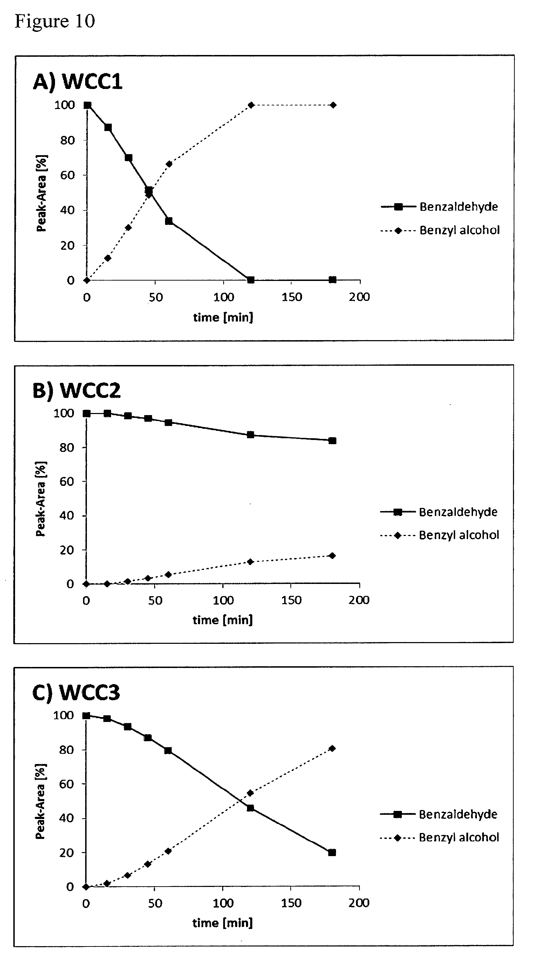

The reduction of benzaldehyde to benzyl alcohol was determined in the whole-cell catalysts WCC1, WCC2 and WCC3 of example 7a). 50 mM of the CF₃-ketone was used as substrate and 5 mg of the whole-cell catalysts WCC1, WCC2 or WCC3, respectively, was used. 500 µM NADP⁺ was added as co-factor. The following reactants were used: 5.05 µl benzaldehyde (= 50 mM), 50 µl methanol, 100 µl WCC (50 mg/ml = 5 mg), 40 µl D-glucose (2.5 M = 100 mM), 5 µl NADP⁺ (0.1 M = 500 µM) and tris HCl buffer (0.5 M pH 7) ad 1 ml. Samples were taken after 0, 15, 30, 45, 60, 120 and 180 minutes and analyzed by gas chromatography. The Figures 10A, 10B and 10C show the reduction of benzaldehyde to benzyl alcohol by the whole-cell catalysts WCC1, WCC2 and WCC3, respectively. From the figures the following whole-cell activities were determined: WCC1: 540 mU (91%), WCC2: 53 mU (54 %) and WCC3: 276 mU (29 %). It can be taken from Figure 10 that the whole-cell catalyst WCC1 almost completely reduces benzaldehyde to benzyl alcohol within 2 hours. Thus, the construct of WCC2 seems the most suitable construct for the reduction of benzaldehyde.

### c) Reaction of the CF₃-ketone with the whole-cell catalysts

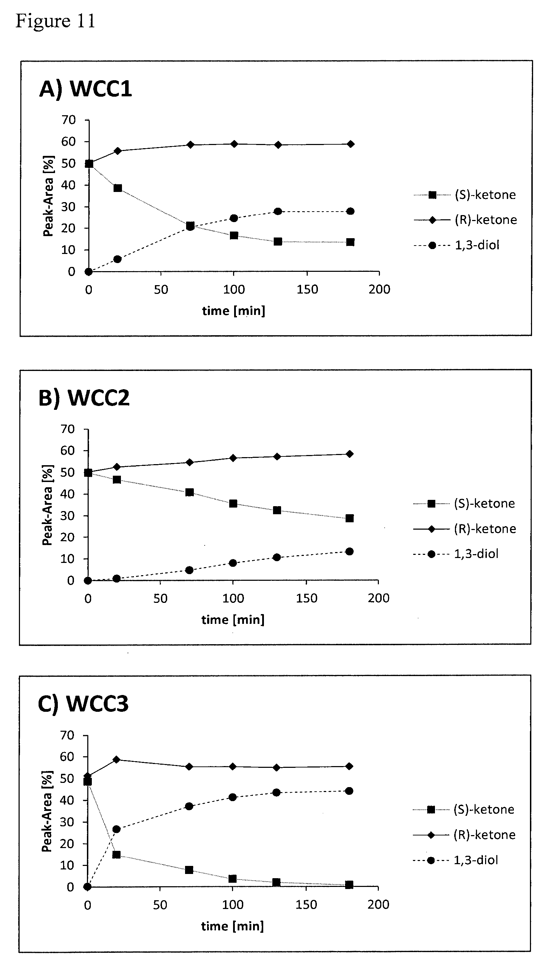

The whole-cell catalysts WCC1, WCC2 and WCC3 further were tested in the enzymatic separation of the racemic the CF₃-ketone. 50 mM of the CF₃-ketone was used as substrate and 5 mg of the whole-cell catalysts WCC1, WCC2 or WCC3, respectively, was used. 500 µM NADP⁺ were added as co-factor. The following reactants were used: 50 µl CF₃-ketone (1 M = 50 mM), 100 ul WCC (50 mg/ml = 5 mg), 20 µl D-glucose (2.5 M = 50 mM), 5 µl NADP⁺ (0.1 M = 500 µM) and tris HCl buffer (0.5 M pH 7) ad 1 ml. Samples were taken after 0, 20, 70, 100, 130 and 180 minutes and analyzed by gas chromatography. The Figures 11 A), 11B) and 11C) show the reduction of the CF₃-ketone by the whole-cell catalysts WCC1, WCC2 and WCC3, respectively. The Figures show the respective peak area for the (S)-ketone, the (R)-ketone, and the alcohol of the (S)-ketone versus time. It can be taken from Figure 11 that the whole-cell catalyst WCC3 almost completely reduces the (S)-ketone to the alcohol within 3 hours and was the most effective of the constructs in the enzymatic racemat resolution. Thus, the construct of WCC3 seems the most suitable construct for the reduction of the CF₃-ketone.

### Example 8: "One-Pot" reaction for the enantioselective synthesis of a (S,S)-1,3-diol

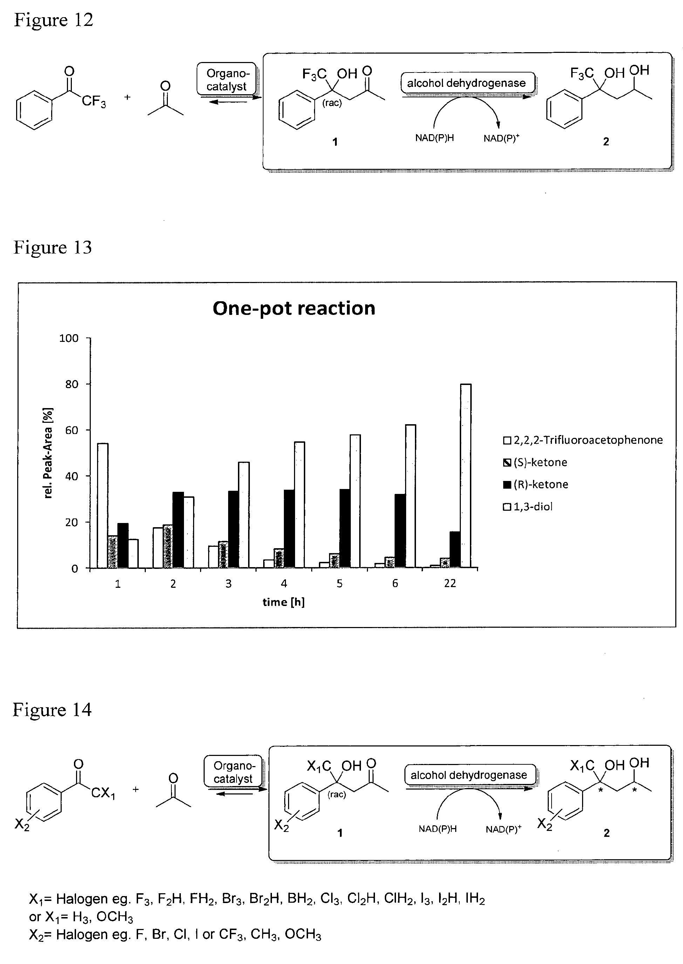

It was tested if a one-pot synthesis combining an enantioselective organo-catalytic aldol reaction of 2,2,2-trifluoroacetophenone and acetone and a diastereoselective, enzymatic reduction of the ketone for the production of chiral 1,3-diols is possible by using the alcohol dehydrogenase from *Pichia pastoris* (PPADH). The scheme depicting the organo-catalytic aldol reaction of 2,2,2-trifluoroacetophenone and acetone to the racemic the CF₃-ketone according to formula 1 followed by the biocatalytic reduction of the CF₃-ketone to a chiral 1,3-diol according to formula 2 having defined stereo chemistry is shown in Figure 12.

For the one-pot synthesis the following reactants were used:
a) for the aldol reaction: 0.1 mmol 2,2,2-trifluoroacetophenone (14 µl), 2.5 eq (equivalents) acetone (18 µl), 1 mol % (*S;S*)-Singh-catalysator (20 mM, 50 µl)
b) for the reduction: 120 µl glucose (2.5 M) = 300 mM, 200 µl fermented WCC3 (50 mg/ml) = 10 mg/ml, 10 µl NADP+ (100 mM) = 1 mM, 588 µl 0.5 M KPi buffer pH 7 in a total volume of 1 ml.

Samples were taken after 1, 2, 3, 4, 5, 2 and 22 hours reaction time and analyzed by gas chromatography. The Figure 13 shows the amount of trifluoroacetophenone, the (S)-ketone, the (R)-ketone, and the 1,3-diol, respectively, given as a percentage of peak area versus time. It can be taken from the Figure 13 that within 22 hours about 80 % diol were synthesized. It is further obvious from Figure 13 that the amount of the (R)-ketone decreased with the reaction time. It could thus be shown that a dynamic kinetic racemate resolution took place as is shown in the Figure 14.

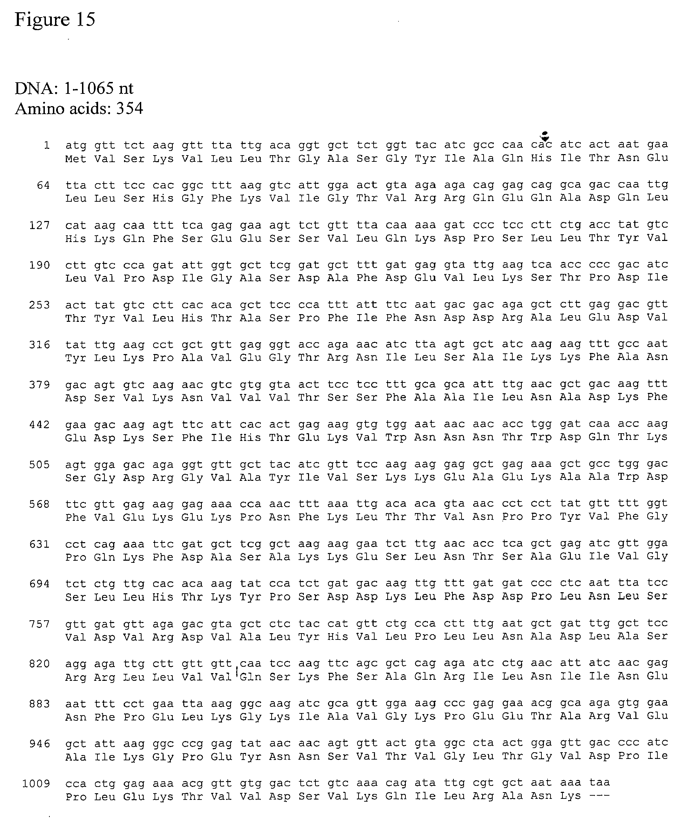

The examples the alcohol dehydrogenase from *Pichia pastoris* (PPADH) whose nucleic acid and protein sequences are shown in Figure 15 was cloned and overexpressed to yield an enzymatically active recombinant protein, which was successfully used for producing diastereomerically pure 1,3-diols. A co-factor regenerating system with glucose dehydrogenase (GDH) also was successfully used for the production of alcohols. The alcohol dehydrogenase from *Pichia pastoris* (PPADH) further was successfully used as a whole-cell catalyst. The alcohol dehydrogenase from *Pichia pastoris* (PPADH) further was successfully used in a one-pot-synthesis comprising an aldol reaction followed by a biocatalytic reduction of the thus produced ketone to a chiral 1,3-diol having defined stereo chemistry by PPADH.

## Claims

1. A nucleic acid coding for an alcohol dehydrogenase according to SEQ ID NO: 1 or the sequence complementary thereto.

2. An alcohol dehydrogenase encoded by a nucleic acid according to Claim 1.

3. An alcohol dehydrogenase from saccharomycetales particularly selected from the group comprising *Pichia pastoris, Wickerhamomyces ciferrii, Cyberlindnera jadinii, Kluyveromyces marxianus, Ogataea parapolymorpha, Kuraishia capsulata, Lachancea quebecensis, Geotrichum candidum, Spathaspora passalidarum, Saccharomyces cerevisiae,* and *Chlamydia trachomatis,* more particularly an alcohol dehydrogenase according to SEQ ID NO:2, or an alcohol dehydrogenase having at least 80% sequence identity to SEQ ID NO: 2.

4. The alcohol dehydrogenase according to claim 3, wherein the alcohol dehydrogenase comprises at least one N-terminal and/or C-terminal His tag, preferably a 6xHis tag HHHHHH (SEQ ID NO: 3).

5. Plasmids and vectors comprising one or more nucleic acids according to claim 1.

6. A microorganism comprising a plasmid according to claim 5.

7. Primer for producing the nucleic acids according to claim 1.

8. Use of the alcohol dehydrogenase according to claims 2 to 4 for producing enantiomer- or diastereomer-enriched organic compounds, such as alcohols, particularly diols.

9. The use of claim 8, wherein producing enantiomer- or diastereomer-enriched organic compounds is performed using a co-factor regenerating system.

10. The use of claim 8 or 9, wherein a co-substrat is used for regenerating the co-factor, preferably a primary or secondary alcohol.

11. Use of the nucleic acid according to claim 1 for producing whole-cell catalysts.

12. A whole-cell catalyst, comprising the nucleic acid according to claim 1.

13. A method for producing diols, the method comprising the step of a reduction of a ketone or an aldehyde catalyzed by the alcohol dehydrogenase according to claims 2 to 4 or the whole-cell catalyst according to claim 12.

14. The method according to claim 13, wherein the method comprises the step of an aldol reaction of acetone and 2,2,2-trifluoroacetophenone or its derivatives, followed by the step of reducing the reaction product catalyzed by the alcohol dehydrogenase according to claims 2 to 4 or a whole-cell catalyst according to claim 12.

15. The method according to claim 13 or 14, wherein the trifluoroacetophenone derivative is selected from the group comprising 4'-chloro-2,2,2-trifluoroacetophenone, 4'-bromo-2,2,2-trifluoroacetophenone, 4'-fluoro-2,2,2-trifluoroacetophenone, 4'-methyl-2,2,2-trifluoroacetophenone, 4'-methoxy-2,2,2-trifluoroacetophenone, 4'-(trifluoromethyl)-2,2,2-trifluoroacetophenone, and 2'-methoxy-2,2,2-trifluoroacetophenone, particularly 2,2,2-trifluoroacetophenone.
